# EUROPEAN PATENT SPECIFICATION

(11) **EP 1 986 661 B1**
(45) Date of publication and mention of the grant of the patent: **29.08.2018**
(21) Application number: 07763560.5
(22) Date of filing: 08.02.2007
(51) Int. Cl.: A61K 31/70, A61K 48/00, C12N 15/864, C12N 9/16, C12N 15/85

(54) **GENE THERAPY FOR NIEMANN-PICK DISEASE TYPE A**
GENTHERAPIE FÜR DIE NIEMANN-PICK-KRANKHEIT VOM TYP A
THERAPIE GENIQUE DESTINEE A LA MALADIE DE NIEMANN-PICK DE TYPE A

(30) Priority: 08.02.2006 US 771628 P; 09.02.2006 US 772360 P
(43) Date of publication of application: 05.11.2008
(73) Proprietor: Genzyme Corporation, Cambridge, MA 02142 (US)
(72) Inventor: PASSINI, Marco, A., Shrewsbury, MA 01545 (US); ZIEGLER, Robin, J., Westford, MA 01886 (US); DODGE, James, Worcester, MA 01603 (US); SHIHABUDDIN, Lamya, Brighton, MA 02135 (US); CHENG, Seng, H., Wellesley, MA 02481 (US)
(74) Representative: Mathys & Squire LLP
(86) International application number: PCT/US2007/003388
(87) International publication number: WO 2007/092563

(56) References cited:
- WO-A2-01/60414
- US-A1- 2004 180 440
- US-A1- 2004 204 379
- US-A1- 2004 258 666
- US-B1- 6 180 613
- DODGE JAMES C ET AL: "Gene transfer of human acid sphingomyelinase corrects neuropathology and motor deficits in a mouse model of Niemann-Pick type A disease", PROCEEDINGS OF THE NATIONAL ACADEMY OF SCIENCES OF THE UNITED STATES OF AMERICA, vol. 102, no. 49, December 2005 (2005-12), pages 17822-17827, XP002671093, ISSN: 0027-8424
- PASSINI M A ET AL: "AAV Vector-Mediated Correction of Brain Pathology in a Mouse Model of Niemann-Pick A Disease", MOLECULAR THERAPY, ACADEMIC PRESS, SAN DIEGO, CA, US, vol. 11, no. 5, 1 May 2005 (2005-05-01), pages 754-762, XP004863183, ISSN: 1525-0016, DOI: 10.1016/J.YMTHE.2005.01.011
- BARBON ET AL: "AAV8-Mediated Hepatic Expression of Acid Sphingomyelinase Corrects the Metabolic Defect in the Visceral Organs of a Mouse Model of Niemann-Pick Disease", MOLECULAR THERAPY, ACADEMIC PRESS, SAN DIEGO, CA, US, vol. 12, no. 3, 1 September 2005 (2005-09-01), pages 431-440, XP005472377, ISSN: 1525-0016, DOI: 10.1016/J.YMTHE.2005.03.011
- "PARTICULARITIES OF LYSOSOMAL STORAGE DISEASES GENE THERAPY", GENE THERAPY, MACMILLAN PRESS LTD., BASINGSTOKE, GB, vol. 3, no. 12, 1 December 1996 (1996-12-01), pages 1039-1041, XP001007386, ISSN: 0969-7128
- JIN H-K ET AL: "Ex vivo gene therapy using bone marrow-derived cells: combined effects of intracerebral and intravenous transplantation in a mouse model of Niemann-Pick disease", MOLECULAR THERAPY, ACADEMIC PRESS, SAN DIEGO, CA, US, vol. 8, no. 6, 1 December 2003 (2003-12-01), pages 876-885, XP003002614, ISSN: 1525-0016
- PASSINI MARCO A ET AL: "Combination brain and systemic injections of AAV provide maximal functional and survival benefits in the Niemann-Pick mouse", PROCEEDINGS OF THE NATIONAL ACADEMY OF SCIENCES OF USA, NATIONAL ACADEMY OF SCIENCE, WASHINGTON, DC; US, vol. 104, no. 22, 29 May 2007 (2007-05-29) , pages 9505-9510, XP002455719, ISSN: 0027-8424, DOI: 10.1073/PNAS.0703509104
- ELLIGER ET AL. MOLECULAR THERAPY vol. 5, no. 5, 2002, pages 617 - 626, XP008090917
- OHASHI T. ET AL. PNAS vol. 94, no. 4, 1997, pages 1287 - 1292, XP002122348
- GONZALEZ-GOMEZ ET AL. AM. J. PATHOLOGY vol. 153, no. 4, 1998, pages 1293 - 1300, XP008090668
- AGUDO ET AL. HUMAN GENE THERAPY vol. 13, no. 5, 20 March 2002, pages 665 - 674, XP003017729
- VOLGER ET AL. J. INHER. METAB. DIS. vol. 21, 1998, pages 575 - 586, XP008091995

## Description

### Related Applications

This application claims priority to U.S. Provisional Application Serial No. 60/771,628, filed February 8, 2006, and U.S. Provisional Application Serial No. 60/772,360 filed February 9, 2006.

### Field of the Invention

The present invention relates to compositions and methods for treating disorders affecting the brain and viscera, e.g., Niemann-Pick Disease Type A.

### Summary of the Invention

A group of metabolic disorders known as lysosomal storage diseases (LSD) includes over forty genetic disorders, many of which involve genetic defects in various lysosomal hydrolases. Representative lysosomal storage diseases and the associated defective enzymes are listed in Table 1.

**Table 1**

| ***Lysosomal storage disease*** | ***Defective enzyme*** |
|---|---|
| Aspartylglucosaminuria | Aspartylglucosaminidase |
| Fabry | A-Galactosidase A |
| Infantile Batten Disease* (CNL1) | Palmitoyl Protein Thioesterase |
| Classic Late Infantile Batten Disease* (CNL2) | Tripeptidyl Peptidase |
| Juvenile Batten Disease* (CNL3) | Lysosomal Transmembrane Protein |
| Batten, other forms* (CNL4-CNL8) | Multiple gene products |
| Cystinosis | Cysteine transporter |
| Farber | Acid ceramidase |
| Fucosidosis | Acid α-L-fucosidase |
| Galactosidosialidosis | Protective protein/cathepsin A |
| Gaucher types 1, 2*, and 3* | Acid β-glucosidase, or glucocerebrosidase |
| G_{M1} gangliosidosis* | Acid β-galactosidase |
| Hunter* | Iduronate-2-sulfatase |
| Hurler-Scheie* | A-L-Iduronidase |
| Krabbe* | Galactocerebrosidase |
| α-Mannosidosis* | Acid α-mannosidase |
| β-Mannosidosis* | Acid β-mannosidase |
| Maroteaux-Lamy | Arylsulfatase B |
| Metachromatic leukodystrophy* | Arylsulfatase A |
| Morquio A | N-Acetylgalactosamine-6-sulfate sulfatase |
| Morquio B | Acid β-galactosidase |
| Mucolipidosis II/III* | N-Acetylglucosamine-1-phosphotransfera se |
| Niemann-Pick A*, B | Acid sphingomyelinase |
| Niemann-Pick C* | NPC-1 |
| Pompe* | Acid α-glucosidase |
| Sandhoff* | β-Hexosaminidase B |
| Sanfilippo A* | Heparan N-sulfatase |
| Sanfilippo B* | A-N-Acetylglucosaminidase |
| Sanfilippo C* | Acetyl-CoA:a-glucosaminide N-acetyltransferase |
| Sanfilippo D* | N-Acetylglucosamine-6-sulfate sulfatase |
| Schindler Disease* | A-N-Acetylgalactosaminidase |
| Schindler-Kanzaki | A-N-Acetylgalactosaminidase |
| Sialidosis | A-Neuramidase |
| Sly* | B-Glucuronidase |
| Tay-Sachs* | B-Hexosaminidase A |
| Wolman* | Acid Lipase |

| | |
|---|---|
| * CNS involvement | |

The hallmark feature of LSD is the abnormal accumulation of metabolites in the lysosomes which leads to the formation of large numbers of distended lysosomes in the perikaryon. A major challenge to treating LSD (as opposed to treating a liver-specific enzymopathy) is the need to reverse lysosomal storage pathology in multiple separate tissues. Some LSDs can be effectively treated by intravenous infusion of the missing enzyme, known as enzyme replacement therapy (ERT). For example, Gaucher type 1 patients have only visceral disease and respond favorably to ERT with recombinant glucocerebrosidase (Cerezyme®, Genzyme Corp.). However, patients with metabolic disease that affects the CNS (e.g., type 2 or 3 Gaucher disease) do not respond completely to intravenous ERT because the replacement enzyme is prevented from entering the brain by the blood brain barrier (BBB). Furthermore, early attempts to introduce a replacement enzyme into the brain by direct injection of the protein have been limited in part due to enzyme cytotoxicity at high local concentrations and limited parenchymal diffusion rates in the brain (Pardridge, Peptide Drug Delivery to the Brain, Raven Press, 1991).

In addition, antibodies may develop against the infused enzymes used in enzyme replacement therapy. Such antibodies may be without clinical significance or may lead to hypersensitivity reactions or decrease bioavalability of the therapeutic proteins. Hunley, T.E. et al. (2004) Pediatrics 114(4):e532-e535. For example, Kakkis E. et al. (2004) PNAS 101 (3):829-834 report that adverse effects of antibodies on enzyme replacement therapy of lysosomal storage disorders has been observed in the canine model of mucopolysaccharidosis I (MPS I). The authors also report on similar results in other animal models of MPS disorders, including MPS I, MPS VI and MPS VII. Reduction of the immune response generated by these proteins may be desirable. The induction of antigen-specific tolerance is a potential method to reduce such an immune response, but has been reported to have been difficult to achieve.

Gene therapy is an emerging treatment modality for disorders affecting the CNS, including LSDs. Promising results in an accepted animal model have been achieved using gene therapy for the treatment of Neimann-Pick Type A disease (NPA). Dodge et al. (2005) PNAS 102(49):18722-17827. NPA is a lysosomal storage disorder caused by a deficiency in acid sphingomyelinase (ASM) activity. Loss of ASM activity results in lysosomal shpingomyelin (SPM) accumulation, secondary metabolic defects such as aberrant cholesterol metabolism, and subsequent loss of cellular function in organ systems including the central nervous system (CNS). Schuchman and Desnick, The Metabolic and Molecular Bases of Inherited Disease, McGraw-Hill, New York, pp. 3589-3610 and Horinouchi et al. (1995) Nat. Genet. 10:288-293.

This disclosure provides a method comprising the steps of administering an effective amount of a viral vector comprising a transgene encoding an immunogen to the mammal's liver tissue and subsequently administering an effective amount of a second viral vector comprising a transgene encoding an immunogen to the mammal's brain.

Also provided is a method for treating Niemann-Pick Type A Disease in a mammal comprising the steps of administering an effective amount of a viral vector comprising a transgene encoding an acid sphingomyelinase polypeptide to the mammal's liver tissue and subsequently administering an effective amount of a second viral vector comprising a transgene encoding an acid sphingomyelinase polypeptide to said mammal's brain, thereby treating Niemann-Pick Type A Disease in the mammal.

The disclosure also provides methods and compositions for tolerizing a mammal's brain to a pre-selected immunogen by first systemically delivering an effective amount of the immunogen via a transgene and then administering an effective amount of the immunogen to the mammal's central nervous system (CNS).

It also provides methods and compositions for tolerizing a mammal's brain to acid sphingomyelinase polypeptide by first delivering an effective amount of a transgene encoding for the polypeptide to the mammal's liver and then administering an effective amount of the transgene for the polypeptide to the mammal's central nervous system (CNS).

The disclosure also provides methods and compositions to ameliorate the symptoms associated with Niemann-Pick Type A disease (NPA) in a mammal suffering from NPA by transducing the mammal's brain tissue with an effective amount of a transgene encoding for acid sphingomyelinase polypeptide after transduction of the mammal's liver with the same transgene. On the basis of the disclosure that is contained herein, in a first aspect the invention provides a first viral vector comprising a transgene encoding an immunogen; and a second viral vector comprising a transgene encoding the immunogen for use in the treatment of lysosomal storage diseases with CNS involvement, wherein said treatment comprises the steps of:
a) administering said first viral vector to a mammal's liver tissue such that the mammal's brain is tolerised to the immunogen; and
b) subsequently administering said second viral vector to said mammal's brain
wherein said subsequent second viral vector administration occurs after said first viral vector comprising a transgene encoding the immunogen has been expressed in the liver of the mammal for an effective amount of time to generate tolerisation, and wherein the immunogen is the defective enzyme associated with the lysosomal storage disease being treated.

In a second aspect, the invention provides a first viral vector comprising a transgene encoding an acid sphingomyelinase polypeptide or protein; and a second viral vector comprising a transgene encoding an acid sphingomyelinase polypeptide or protein for use in the treatment of Niemann-Pick Type A disease in a mammal comprising the steps of:
a) administering said first viral vector to said mammal's liver tissue; and
b) subsequently administering said second viral vector to said mammal's brain,
wherein said subsequent second viral vector administration occurs after said first viral vector comprising a transgene encoding an an acid sphingomyelinase polypeptide or protein has been expressed in the liver of the mammal for an effective amount of time to generate tolerisation.

Additional aspects and embodiments of the present invention are set forth in the appended claims.

Additional advantages of the invention and disclosure will be set forth in part in the following description, and in part will be understood from the description, or may be learned by practice of the invention.

### Brief Description of the Figures

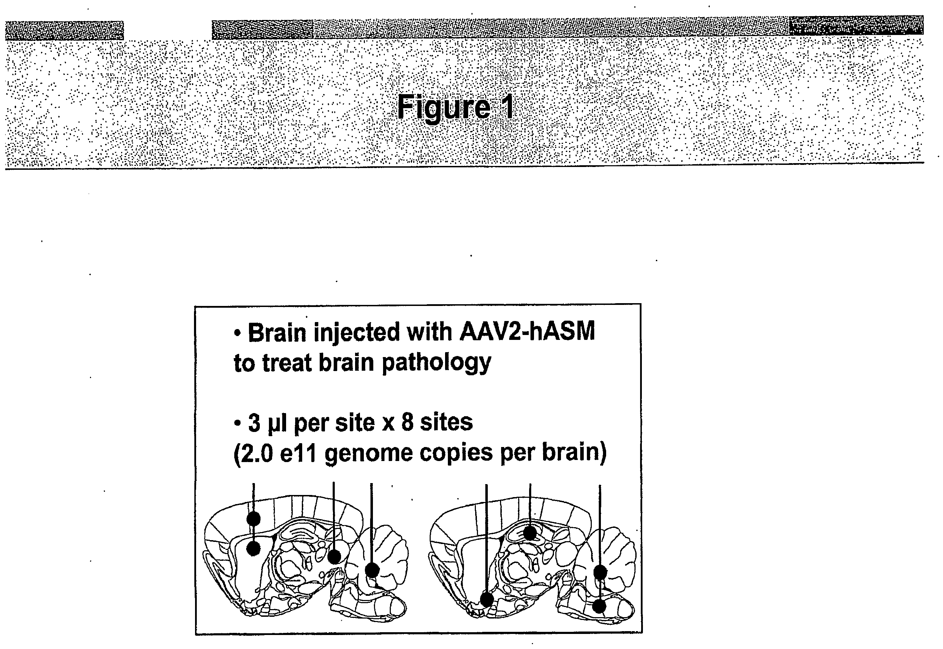

Figure 1 graphically shows various administration sites of the transgene into the central nervous systems of the mice.

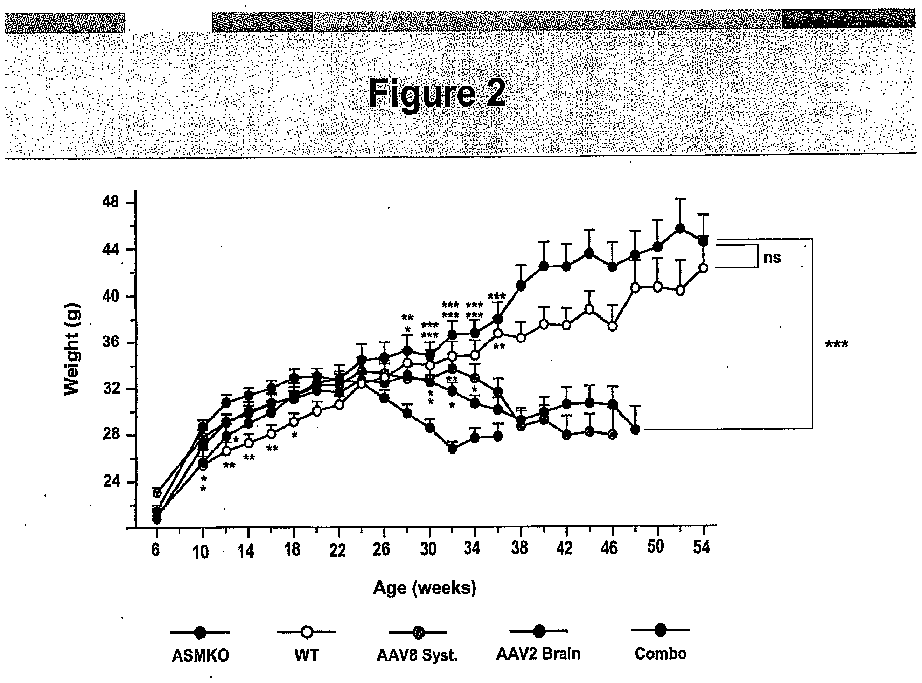

Figure 2 graphically shows body weight of the treated mice as a measure of fitness. Measurements began at the age of 6 weeks (treatment began at week four). During the time period of 6 to 36 weeks (2 to 32 weeks after systemic injection of the transgene) all groups were compared to untreated ASMKO mice. Figure legend: *p < 0.05; **p < 0.01; ***p < 0.001; ns (not significant).

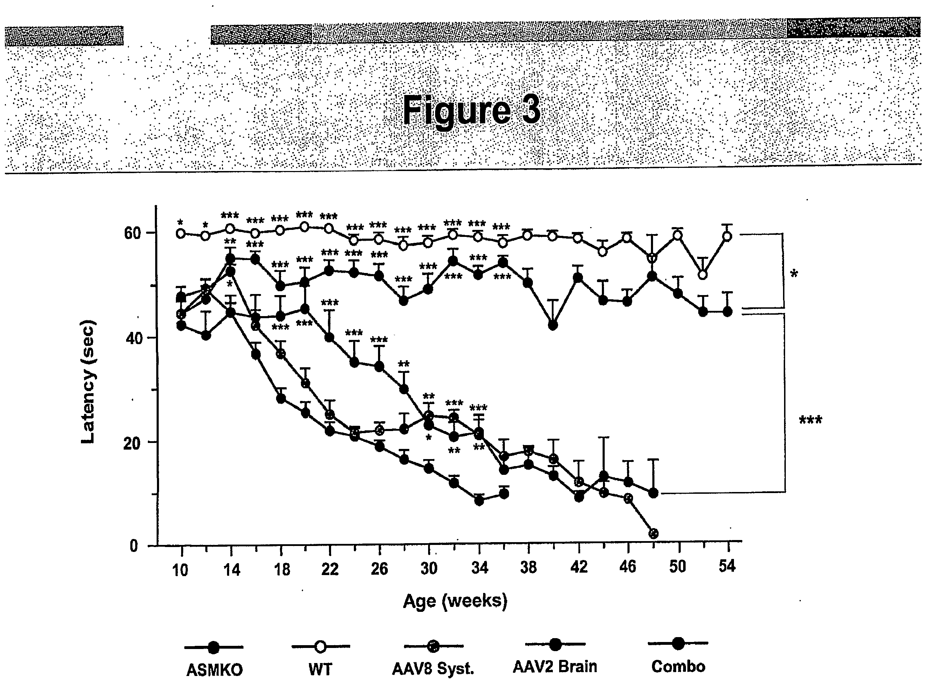

Figure 3 graphically shows the results of Accelerating Rotarod test as a measurement of recovery of motor function. Measurements began at the age of 10 weeks (treatment began at week four). During the time period between 10 and 36 weeks (6 to 32 weeks after systemic injection of the transgene) all groups were compared to untreated ASMKO mice. Figure legend: *p < 0.05; **p < 0.01; ***p < 0.001; ns (not significant).

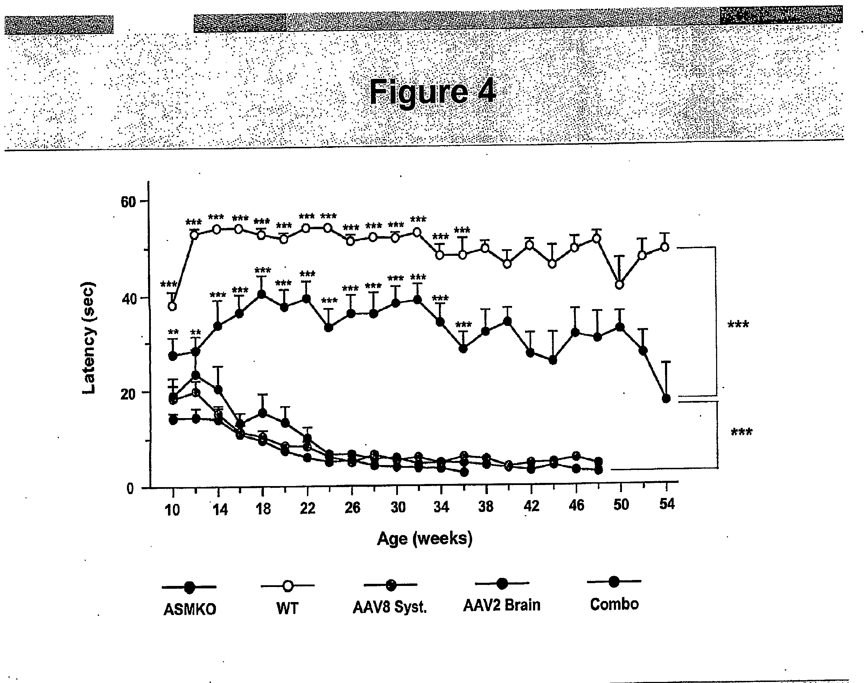

Figure 4 graphically shows the results of Rocking Rotarod test as a measurement of recovery of motor function. Measurements began at the age of 10 weeks (treatment began at week four). During the time period between 10 and 36 weeks (6 to 32 weeks after systemic injection of the transgene) all groups were compared to untreated ASMKO mice. Figure legend: *p < 0.05; **p < 0.01; ***p < 0.001; ns (not significant).

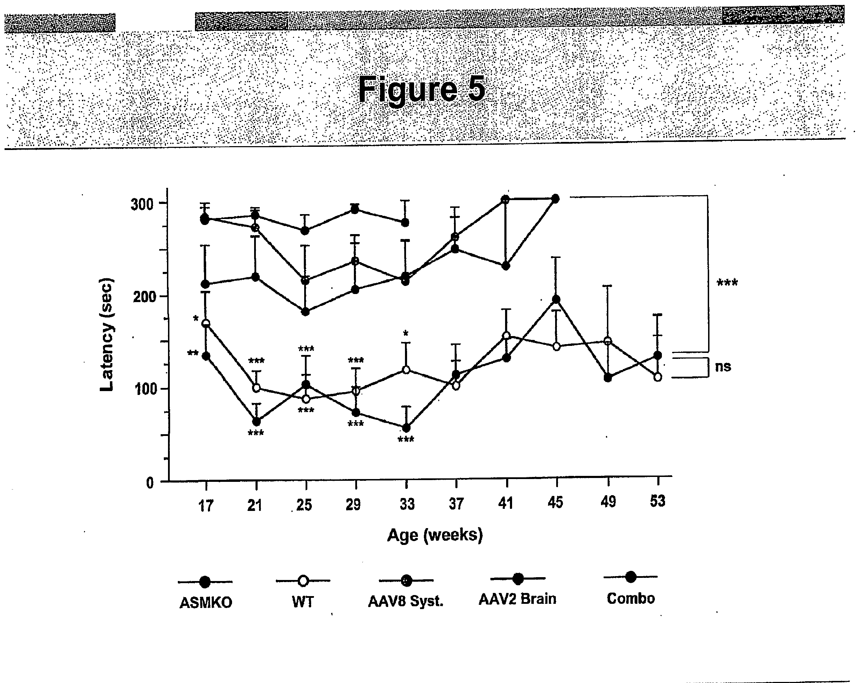

Figure 5 graphically shows the results of Barnes Maze test as a measurement of recovery of cognitive function. Measurements began at the age of 17 weeks (treatment began at week four). During the time period between 17 and 33 weeks (13 to 29 weeks after systemic injection of the transgene) all groups were compared to untreated ASMKO mice. Figure legend: *p < 0.05; **p < 0.01; ***p < 0.001; ns (not significant).

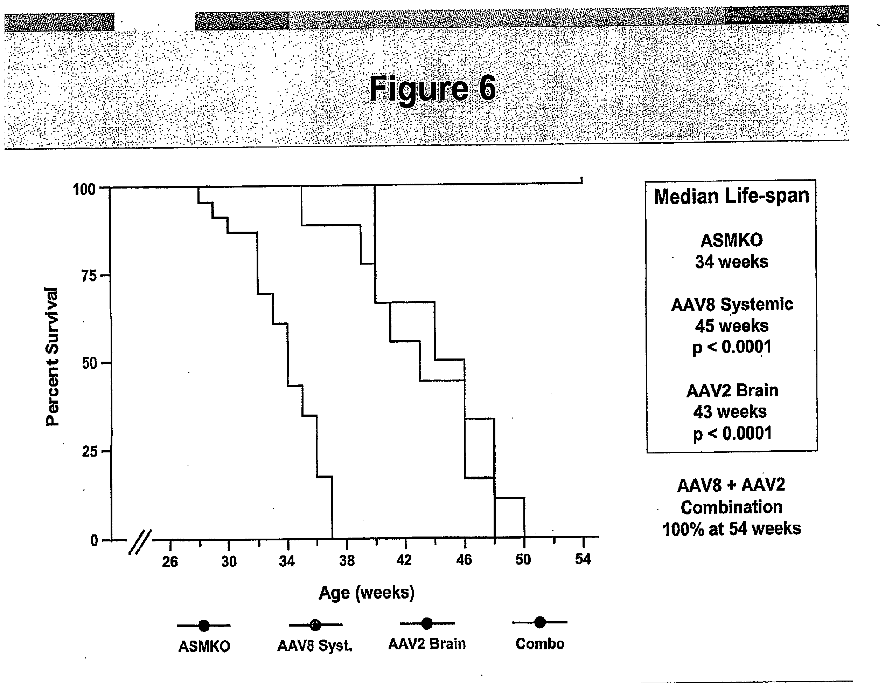

Figure 6 graphically shows that ASM combination gene therapy extends the life span of ASMKO mice. Median life span of ASMKO mice was 34 weeks. Median life span for mice receiving systemic transgene was 45 weeks (p < 0.0001). Median life span for mice receiving intracranial transgene was 43 weeks (p < 0.0001). Life span for mice receiving intracranial and systemic transgene therapy was 100 % at 54 weeks.

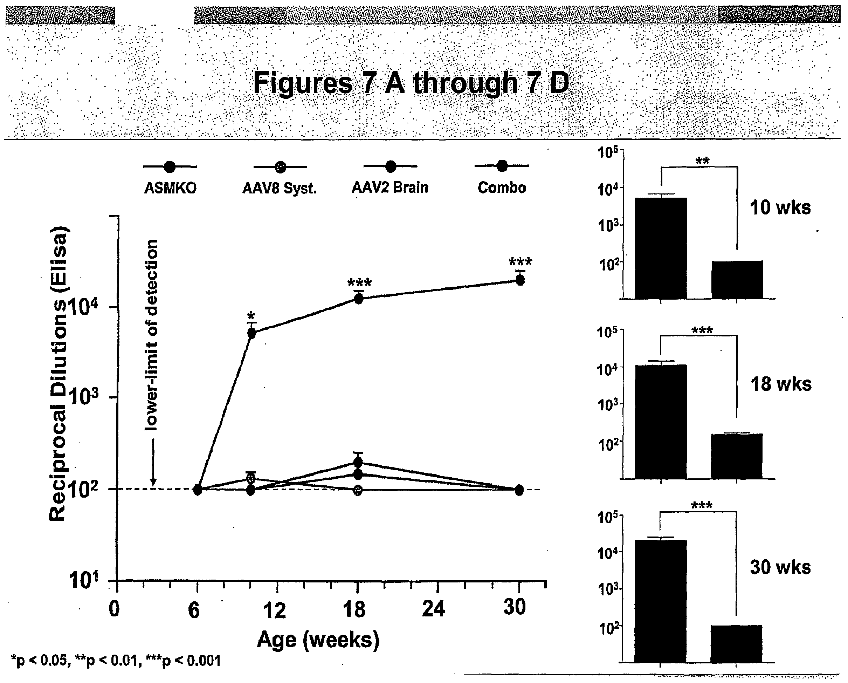

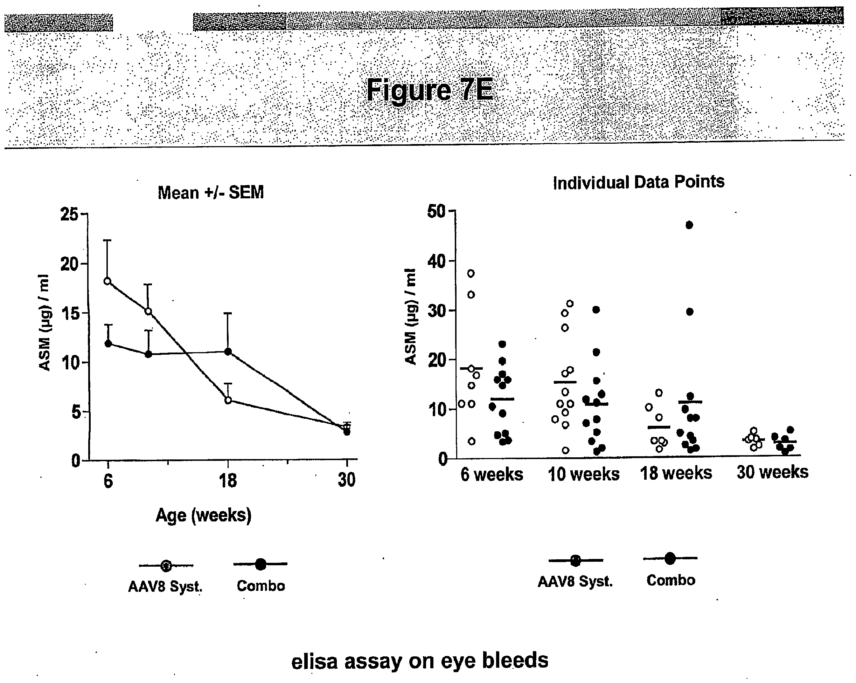

Figures 7 A through 7 D graphically show levels of anti-hASM antibodies in circulation for treated and untreated mice. Figure legend: *p < 0.05; **p < 0.01; ***p < 0.001. Figure 7 E shows human ASM protein levels in blood serum over time.

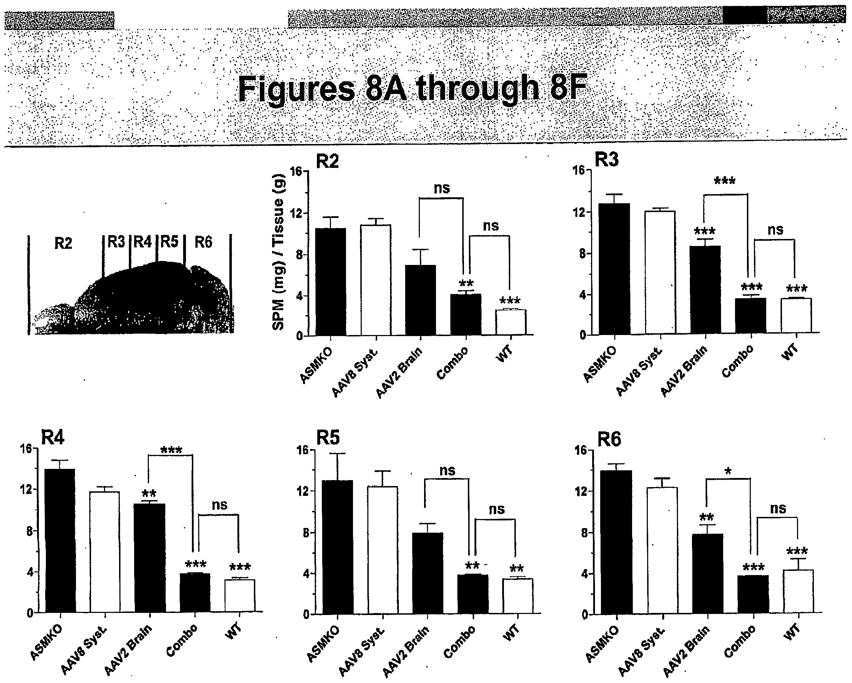

Figures 8 A through 8 F graphically show sphingomyelin levels in the brain of treated and untreated mice.

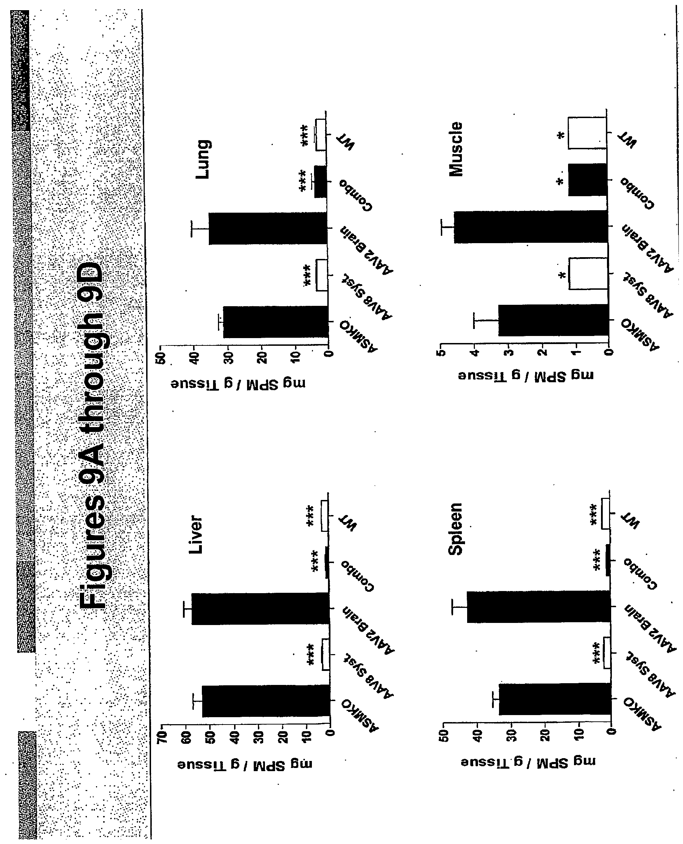

Figures 9 A through 9 D graphically show sphingomyelin levels in various viscera of treated and untreated mice.

### Detailed Description of The Invention

Throughout this disclosure, various publications, patents and published patent specifications are referenced by an identifying citation.

### Definitions

The practice of the present invention will employ, unless otherwise indicated, conventional techniques of immunology, molecular biology, microbiology, cell biology and recombinant DNA, which are within the skill of the art. See e.g., Sambrook, Fritsch and Maniatis, Molecular Cloning: A Laboratory Manual, 2nd edition (1989); Current Protocols In Molecular Biology (F. M. Ausubel et al. eds., (1987)); the series Methods In Enzymology (Academic Press, Inc.): Pcr 2: A Practical Approach (M.J. MacPherson, B.D. Hames and G.R. Taylor eds. (1995)), Harlow and Lane, eds. (1988) ANTIBODIES, A LABORATORY MANUAL And ANIMAL CELL CULTURE (R.I. Freshney, ed. (1987)).

As used herein, certain terms have the following defined meanings. As used in the specification and claims, the singular form "a", "an" and "the" include plural references unless the context clearly dictates otherwise. For example, the term "a cell" includes a plurality of cells, including mixtures thereof.

The terms "polynucleotide" and "oligonucleotide" are used interchangeably and refer to a polymeric form of nucleotides of any length, either deoxyribonucleotides or ribonucleotides or analogs thereof. Polynucleotides can have any three-dimensional structure and may perform any function, known or unknown. The following are non-limiting examples of polynucleotides: a gene or gene fragment (for example, a probe, primer, EST or SAGE tag), exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes and primers. A polynucleotide can comprise modified nucleotides, such as methylated nucleotides and nucleotide analogs. If present, modifications to the nucleotide structure can be imparted before or after assembly of the polymer. The sequence of nucleotides can be interrupted by non-nucleotide components. A polynucleotide can be further modified after polymerization, such as by conjugation with a labeling component. The term also refers to both double- and single-stranded molecules. Unless otherwise specified or required, any instance of this disclosure that is a polynucleotide encompasses both the double-stranded form and each of two complementary single-stranded forms known or predicted to make up the double-stranded form.

A polynucleotide is composed of a specific sequence of four nucleotide bases: adenine (A); cytosine (C); guanine (G); thymine (T); and uracil (U) for guanine when the polynucleotide is RNA. Thus, the term "polynucleotide sequence" is the alphabetical representation of a polynucleotide molecule. This alphabetical representation can be input into databases in a computer having a central processing unit and used for bioinformatics applications such as functional genomics and homology searching.

A "gene" refers to a polynucleotide containing at least one open reading frame (ORF) that is capable of encoding a particular polypeptide or protein after being transcribed and translated. Any of the polynucleotides sequences described herein may be used to identify larger fragments or full-length coding sequences of the gene with which they are associated. Methods of isolating larger fragment sequences are known to those of skill in the art.

A "gene product" or alternatively a "gene expression product" refers to the amino acid (e.g., peptide or polypeptide) generated when a gene is transcribed and translated.

The term "polypeptide" is used interchangeably with the term "protein" and in its broadest sense refers to a compound of two or more subunit amino acids, amino acid analogs or peptidomimetics. The subunits may be linked by peptide bonds. In another embodiment, the subunit may be linked by other bonds, e.g., ester, ether, etc. As used herein the term "amino acid" refers to either natural and/or unnatural or synthetic amino acids, including glycine and both the D and L optical isomers, amino acid analogs and peptidomimetics. A peptide of three or more amino acids is commonly called an oligopeptide if the peptide chain is short. If the peptide chain is long, the peptide is commonly called a polypeptide or a protein.

"Under transcriptional control" is a term well understood in the art and indicates that transcription of a polynucleotide sequence, usually a DNA sequence, depends on its being operatively linked to an element which contributes to the initiation of, or promotes, transcription. "Operatively linked" refers to a juxtaposition wherein the elements are in an arrangement allowing them to function.

0As used herein, the term "comprising" is intended to mean that the compositions and methods include the recited elements, but not excluding others. "Consisting essentially of" when used to define compositions and methods, shall mean excluding other elements of any essential significance to the combination. Thus, a composition consisting essentially of the elements as defined herein would not exclude trace contaminants from the isolation and purification method and pharmaceutically acceptable carriers, such as phosphate buffered saline, preservatives and the like. "Consisting of shall mean excluding more than trace elements of other ingredients and substantial method steps for administering the compositions of this invention. Embodiments defined by each of these transition terms are within the scope of this invention.

The term "isolated" means separated from constituents, cellular and otherwise, in which the polynucleotide, peptide, polypeptide, protein, antibody or fragment(s) thereof, are normally associated with in nature. In one aspect of this disclosure, an isolated polynucleotide is separated from the 3' and 5' contiguous nucleotides with which it is normally associated with in its native or natural environment, e.g., on the chromosome. As is apparent to those of skill in the art, a non-naturally occurring polynucleotide, peptide, polypeptide, protein, antibody or fragment(s) thereof, does not require "isolation" to distinguish it from its naturally occurring counterpart. In addition, a "concentrated", "separated" or "diluted" polynucleotide, peptide, polypeptide, protein, antibody or fragment(s) thereof, is distinguishable from its naturally occurring counterpart in that the concentration or number of molecules per volume is greater than "concentrated" or less than "separated" than that of its naturally occurring counterpart. A polynucleotide, peptide, polypeptide, protein, antibody or fragment(s) thereof, which differs from the naturally occurring counterpart in its primary sequence or for example, by its glycosylation pattern, need not be present in its isolated form since it is distinguishable from its naturally occurring counterpart by its primary sequence or, alternatively, by another characteristic such as glycosylation pattern. Thus, a non-naturally occurring polynucleotide is provided as a separate instance from the isolated naturally occurring polynucleotide. A protein produced in a bacterial cell is provided as a separate instance from the naturally occurring protein isolated from a eukaryotic cell in which it is produced in nature.

"Gene delivery," "gene transfer," and the like as used herein, are terms referring to the introduction of an exogenous polynucleotide (sometimes referred to as a "transgene") into a host cell, irrespective of the method used for the introduction. Such methods include a variety of well-known techniques such as vector-mediated gene transfer (by, e.g., viral infection/transfection or various other protein-based or lipid-based gene delivery complexes) as well as techniques facilitating the delivery of "naked" polynucleotides (such as electroporation, "gene gun" delivery and various other techniques used for the introduction of polynucleotides). The introduced polynucleotide may be stably or transiently maintained in the host cell. Stable maintenance typically requires that the introduced polynucleotide either contains an origin of replication compatible with the host cell or integrates into a replicon of the host cell such as an extrachromosomal replicon (e.g., a plasmid) or a nuclear or mitochondrial chromosome. A number of vectors are known in the art to be capable of mediating transfer of genes to mammalian cells.

A "gene delivery vehicle" is defined as any molecule that can carry inserted polynucleotides into a host cell. Examples of gene delivery vehicles are liposomes, biocompatible polymers, including natural polymers and synthetic polymers; lipoproteins; polypeptides; polysaccharides; lipopolysaccharides; artificial viral envelopes; recombinant yeast cells, metal particles; and bacteria or viruses, such as baculovirus, adenovirus and retrovirus, bacteriophage, cosmid, plasmid, fungal vectors and other recombination vehicles typically used in the art which have been described for expression in a variety of eukaryotic and prokaryotic hosts and may be used for gene therapy as well as for simple protein expression.

A "viral vector" is defined as a recombinantly produced virus or viral particle that comprises a polynucleotide to be delivered into a host cell, either *in vivo, ex vivo* or *in vitro.* Examples of viral vectors include retroviral vectors, adenovirus vectors, adeno-associated virus vectors, alphavirus vectors and the like. Alphavirus vectors, such as Semliki Forest virus-based vectors and Sindbis virus-based vectors, have also been developed for use in gene therapy and immunotherapy. See, Schlesinger and Dubensky (1999) Curr. Opin. Biotechnol. 5:434-439 and Ying et al. (1999) Nat. Med. 5(7):823-827. In aspects where gene transfer is mediated by a retroviral vector, a vector construct refers to the polynucleotide comprising the retroviral genome or part thereof and a therapeutic gene. As used herein, "retroviral mediated gene transfer" or "retroviral transduction" carries the same meaning and refers to the process by which a gene or nucleic acid sequences are stably transferred into the host cell by virtue of the virus entering the cell and integrating its genome into the host cell genome. The virus can enter the host cell via its normal mechanism of infection or be modified such that it binds to a different host cell surface receptor or ligand to enter the cell. As used herein, "retroviral vector" refers to a viral particle capable of introducing exogenous nucleic acid into a cell through a viral or viral-like entry mechanism.

In aspects where gene transfer is mediated by a DNA viral vector, such as an adenovirus (Ad) or adeno-associated virus (AAV), a vector construct refers to the polynucleotide comprising the viral genome or part thereof and a transgene. Adenoviruses (Ads) are a relatively well characterized, homogenous group of viruses, including over 50 serotypes. See e.g., WO 95/27071. Ads are easy to grow and do not require integration into the host cell genome. Recombinant Ad derived vectors, particularly those that reduce the potential for recombination and generation of wild-type virus, have also been constructed. See e.g., WO 95/00655 and WO 95/11984. Wild-type AAV has high infectivity and specificity integrating into the host cell's genome. See, Hermonat and Muzyczka (1984) Proc. Natl. Acad. Sci. USA 81:6466-6470 and Lebkowski et al. (1988) Mol. Cell. Biol. 8:3988-3996. Recombinant AAV vectors are also known in the art. See, e.g., WO 01/36620 A2.

Vectors that contain both a promoter and a cloning site into which a polynucleotide can be operatively linked are well known in the art. Such vectors are capable of transcribing RNA *in vitro* or *in vivo* and are commercially available from sources such as Stratagene (La Jolla, CA) and Promega Biotech (Madison, WI). In order to optimize expression and/or *in vitro* transcription, it may be necessary to remove, add or alter 5' and/or 3' untranslated portions of the clones to eliminate extra, potential inappropriate alternative translation initiation codons or other sequences that may interfere with or reduce expression, either at the level of transcription or translation. Alternatively, consensus ribosome binding sites can be inserted immediately 5' of the start codon to enhance expression.

Gene delivery vehicles also include several non-viral vectors, including DNA/liposome complexes, recombinant yeast cells and targeted viral protein-DNA complexes. Liposomes that also comprise a targeting antibody or fragment thereof can be used in the methods of this disclosure. To enhance delivery to a cell, the nucleic acid or proteins of this invention can be conjugated to antibodies or binding fragment(s) thereof which bind cell surface antigens, e.g., TCR, CD3 or CD4.

The terms "genome particles (gp)," or "genome equivalents," as used in reference to a viral titer, refer to the number of virions containing the recombinant AAV DNA genome, regardless of infectivity or functionality. The number of genome particles in a particular vector preparation can be measured by procedures such as described in the Examples herein, or for example, in Clark et al. (1999) Hum. Gene Ther. 10:1031-1039 and Veldwijk et al. (2002) Mol. Ther. 6:272-278.

The terms "infection unit (iu)," "infectious particle," or "replication unit," as used in reference to a viral titer, refer to the number of infectious vector particles as measured by the infectious center assay, also known as replication center assay, as described, for example, in McLaughlin et al. (1988) J. Virol. 62: 1963-1973.

The term "transducing unit (tu)" as used in reference to a viral titer, refers to the number of infectious vector particles that result in the production of a functional transgene product as measured in functional assays such as described in in Xiao et al. (1997) Exp. Neurobiol. 144:113-124 or in Fisher et al. (1996) J. Virol. 70:520-532 (LFU assay).

The terms "therapeutic," "therapeutically effective amount," and their cognates refer to that amount of a compound that results in prevention or delay of onset or amelioration of symptoms of in a subject or an attainment of a desired biological outcome, such as correction of neuropathology, e.g., cellular pathology associated with a lysosomal storage disease such as that described herein or in Walkley (1998) Brain Pathol. 8:175-193. The term "therapeutic correction" refers to that degree of correction that results in prevention or delay of onset or amelioration of symptoms in a subject. The effective amount can be determined by methods well-known in the art and as described in the subsequent sections.

A "composition" is intended to mean a combination of active agent and another compound or composition, inert (for example, a detectable agent or label) or active, such as an adjuvant.

A "pharmaceutical composition" is intended to include the combination of an active agent with a carrier, inert or active, making the composition suitable for diagnostic or therapeutic use *in vitro, in vivo* or *ex vivo.*

As used herein, the term "pharmaceutically acceptable carrier" encompasses any of the standard pharmaceutical carriers, such as a phosphate buffered saline solution, water and emulsions, such as an oil/water or water/oil emulsion and various types of wetting agents. The compositions also can include stabilizers and preservatives. For examples of carriers, stabilizers and adjuvants; see, Martin, Remington's Pharm. Sci., 15th Ed. (Mack Publ. Co., Easton (1975)).

An "effective amount" is an amount sufficient to effect beneficial or desired results such as prevention or treatment. An effective amount can be administered in one or more administrations, applications or dosages.

A "subject," "individual" or "patient" is used interchangeably herein, which refers to a vertebrate, preferably a mammal, more preferably a human. Mammals include, but are not limited to, murines, simians, humans, farm animals, sport animals and pets.

A "control" is an alternative subject or sample used in an experiment for comparison purpose. A control can be "positive" or "negative". For example, where the purpose of the experiment is to determine a correlation of an altered expression level of a gene with a disease, it is generally preferable to use a positive control (a subject or a sample from a subject, carrying such alteration and exhibiting syndromes characteristic of that disease) and a negative control (a subject or a sample from a subject lacking the altered expression and clinical syndrome of that disease).

Administration of proteins via gene therapy of viral vectors capable of infecting post-mitotic neurons. For a review of viral vectors for gene delivery to the CNS, see Davidson et al. (2003) Nature Rev. 4:353-364. Adeno-associated virus (AAV) vectors are useful for CNS gene therapy because they are substantially non-toxic, non-immunogenic, neurotropic and can sustain expression in the CNS (Kaplitt et al. (1994) Nat. Genet. 8:148-154; Bartlett et al. (1998) Hum. Gene Ther. 9:1181-1186; and Passini et al. (2002) J. Neurosci., 22:6437-6446). As demonstrated herein expression of a transgene in the brain of an animal resulted in an immune response to the transgene product. See Figure 7A.

Applicants have discovered that administration of a transgene to the CNS of a mammal that has been tolerized to the transgene increases efficacy of the treatment. Thus, in one aspect, this disclosure provides a method to tolerize a mammal's brain to a pre-selected immunogen by systemically administering an effective amount of the pre-selected immunogen to the mammal prior to administration of an effective amount of the pre-selected immunogen to the mammal's CNS. As used herein, the term "tolerize" is intended to mean reduce the immune response to an immunogen. The term "immunogen" shall include any agent that initially raises an immune response (T cell or B cell mediated). As demonstrated by Ziegler et al. (2004), transgene expression in the liver following administration of a recombinant AAV vector encoding for the transgene under the control of a liver-specific enhancer/promoter resulted in a reduced antibody response against the expressed transgene.

The method is suitable for any mammal, and as such, a "mammal" includes, but is not limited to murines, simians, humans, farm animals, sport animals and pets. In one particular aspect, the mammal is the ASKMO mouse which is the accepted animal model types A and B Niemann-Pick disease. Horinouchi et al. (1995) Nat. Genetics 10:288-293; Jin et al. (2002) J. Clin. Invest. 109:1183-1191; and Otterbach (1995) Cell 81:1053-1061.

Niemann-Pick A disease (NPA) is classified as a lysosomal storage disease and is an inherited neurometabolic disorder characterized by a genetic deficiency in acid sphingomyelinase (ASM; sphingomyelin cholinephosphohydrolase, EC 3.1.3.12). The lack of functional ASM protein results in the accumulation of sphingomyelin substrate within the lysosomes of neurons and glia throughout the brain. This leads to the formation of large numbers of distended lysosomes in the perikaryon, which are a hallmark feature and the primary cellular phenotype of type A NPD. The presence of distended lysosomes correlates with the loss of normal cellular function and a progressive neurodegenerative course that leads to death of the affected individual in early childhood (The Metabolic and Molecular Bases of Inherited Diseases, eds. Scriver et al., McGraw-Hill, New York, 2001, pp. 3589-3610). Secondary cellular phenotypes (e.g., additional metabolic abnormalities) are also associated with this disease, notably the high level accumulation of cholesterol in the lysosomal compartment. Sphingomyelin has strong affinity for cholesterol, which results in the sequestering of large amounts of cholesterol in the lysosomes of ASMKO mice and human patients. Leventhal et al. (2001) J. Biol. Chem., 276:44976-44983; Slotte (1997) Subcell. Biochem. 28:277-293; and Viana et Ia. (1990) J. Med. Genet. 27:499-504.

In one specific aspect, the systemic site of administration is the mammal's liver. Any method of administration can be used, examples of which are provided below.

After systemic administration of the transgene, it is administered to the CNS of the mammal and in particular, intracranially directly into the mammal's brain and more particularly at a site selected from the group consisting of the brainstem, the hippocampus, the striatum, the medulla, the pons, the mesencephalon, the cerebellum, the midbrain, the thalamus, the hypothalamus, the cerebral cortex, the occipital lobe, the temporal lobe, the parietal lobe, and the frontal lobe. In one embodiment, the administration is specific to the deep cerebellar nuclei of the cerebellum.

As noted above, the transgene encoding the polypeptide or protein is administered to the mammal to ultimately deliver the polypeptide or protein using any appropriate gene transfer methods, examples of which are described supra and in U.S. Patent No. 6,066,626. In one aspect, the transgene encodes ASM. The genomic and functional cDNA sequences of human ASM have been published in for example, U.S. Patent Nos.: 5,773,278 and 6,541,218). Other suitable protein immunogens having CNS involvement are identified in Table 1.

Viral vectors are useful gene transfer vectors. In a particular embodiment, the viral vector is selected from the group consisting of adenovirus, adeno associated virus (AAV), vaccinia, herpesvirus, baculovirus and retrovirus.

Suitable AAV vectors are described throughout U.S. Patent No. 6,066,626 and PCT Publication No. WO 01/36620 A2. Modified vectors, as described in column 9, lines 14 to 66, can also be used. Suitable serotypes include, but are not limited to AAV1, AAV2, AAV3, AAV4, AAV5, AAV6, AAV7 or AAV8. The AAV vector may be a recombinant or a hybrid AAV vector, e.g., one selected from the group consisting of AAV2/1, AAV2/5, AAV2/7, AAV2/8, AAV1/2, AAV1/3, AAV1/5, AAV1/7 and AAV1/8, serotype vectors, wherein the nomenclature refers to the serotype from which the ITRs are generated/the serotype from which the capsid is generated. For example, an AAV2/5 vector comprises the AAV2 serotype ITRs and the AAV5 serotype capsid.

An effective amount of the polypeptide, by administration of a viral vector comprising a transgene from which the polypeptide is generated, is delivered to the mammal. In one aspect, a viral vector comprising a transgene is delivered to the liver and, immediately subsequent to liver delivery, a viral vector comprising said transgene is delivered to the CNS.

In an alternate instance, the subsequent viral vector administration to the CNS occurs after the polypeptide has been expressed in the mammal for an effective amount of time to generate tolerization of the mammal to said polypeptide. This may vary with the patient being treated, the polypeptide delivered, and the desired therapeutic effect. The appropriate time course and number of subsequent administrations to the CNS is determined by the treating physician. To determine whether a mammal is tolerized to a polypeptide, the mammal may be challenged with the polypeptide to determine if such a challenge generates an immune response against the polypeptide. The immune response may be determined by measuring an antibody titer against the polypeptide after the challenge. A reduced or insignificant antibody titer, as compared to the appropriate controls, following challenge may be indicative of a tolerized state. Means to measure and generate antibody responses in mammals, to challenge a mammal with an antigen or immunogen, and to determine antibody titers are well-known in the art. The appropriate amount of time to generate tolerization of the mammal may also be selected by determining the effective amount of time for expression to generate such tolerization in a test subject. The selected amount of time may then be applied to the instant methods without the need to test each patient individually.

In one instance, the subsequent administration of the viral vector to the CNS occurs after expression of the polypeptide encoded by the viral vector delivered to the liver is detected. Detection of the polypeptide's expression can be accomplished by any method known in the art, examples of which include, but are not limited to molecularly (by detecting mRNA) or immunologically (by detecting protein expression) or biochemically (by detecting polypeptide activity, such as enzymatic activity, where such an activity exists).

Alternatively, the subsequent administration can be hours or days or weeks subsequent to the first administration. The use of multiple CNS administrations to multiple CNS administration sites are also within the scope of this disclosure.

Thus, in a specific aspect of this disclosure, a method to tolerize a mammal's brain to acid sphingomyelinase polypeptide is provided. The method systemically administers an effective amount of a transgene encoding the polypeptide to the mammal's liver prior to administration of an effective amount of the transgene encoding the polypeptide to the mammal's central nervous system tissue (CNS).

In a further particular aspect of this disclosure, a method to ameliorate the symptoms associated with Niemann-Pick Type A disease (NPA) in a mammal suffering from NPA is provided. This method requires administering to the mammal's central nervous system (CNS) an effective amount of transgene encoding an acid sphingomyelinase polypeptide and wherein said administration is subsequent to systemic administration of an effective amount of the transgene to the mammal's liver tissue, such that the mammal's ability to raise an antigen-specific immune response to the polypeptide is abrogated or significantly reduced prior to the CNS administration.

In another aspect, the disclosure provides a method to ameliorate the symptoms associated with Niemann-Pick Type A disease (NPA) in a mammal suffering from NPA. Such symptoms include, but are not limited to weight loss or cachexia, loss of motor function, loss of cognitive function and premature death. The method requires administering to the mammal's CNS an effective amount of a viral vector comprising a transgene encoding acid sphingomyelinase polypeptide after administration of an effective amount of a viral vector comprising said transgene to the mammal's liver tissue. In another instance , the area of the CNS to which the viral vector is delivered is the brain.

In a yet further aspect, the disclosure provides a method to treat Niemann-Pick Type A disease (NPA) in a mammal suffering from NPA by administering an effective amount of an AAV viral vector comprising a transgene encoding an acid sphingomyelinase polypeptide to the mammal's liver tissue and subsequently delivering an effective amount of an AAV vector comprising a transgene encoding an acid sphingomyelinase polypeptide to the mammal's CNS, thereby treating NPA in the mammal. In another instance of the disclosure, and in administration of the second viral vector according to the claimed invention, the area of the CNS to which the viral vector is delivered is the brain.

As used herein, the terms "treating," "treatment" and the like are used herein to mean obtaining a desired therapeutic, pharmacologic and/or physiologic effect. The effect may be prophylactic in terms of completely or partially preventing the disease or a sign or symptom thereof, and/or may be therapeutic in terms of a partial or complete cure for the disorder and/or adverse effect attributable to the disorder.

"Treating" also covers any treatment of a disorder in a mammal, and includes: preventing a disorder from occurring in a subject that may be predisposed to a disorder, but has not yet been diagnosed as having it, e.g., a patient who has tested positive for the genetic marker for the disease; inhibiting a disorder, i.e., arresting its development; or relieving or ameliorating the disorder, e.g., cause regression of the disorder, e.g., NPA disease.

As used herein, to "treat" further includes systemic amelioration of the symptoms associated with the pathology and/or a delay in onset of symptoms. Clinical and sub-clinical evidence of "treatment" will vary with the pathology, the individual and the treatment.

In some instances , the method comprises administration of an AAV vector carrying a pre-selected immunogen or transgene so that the transgene product is expressed at a therapeutic level in the selected site. In some instances and embodiments, the viral titer of the composition is at least: (a) 1.5, 2.0, 2.25, 2.5, 2.75, 3.0, 3.25, 3.5, 4.0, 4.5 5, 6, 7, 8, 8.4, 9, 9.3, 10, 15, 20, 25, or 50 (×10¹¹ genome copies per injection (gc); (b) 1.5, 2.0, 2.25, 2.5, 2.75, 3.0, 3.25, 3.5, 4.0, 4.5, 5, 6, 7, 8, 8.4, 9, 9.3, 10, 15, 20, 25, or 50 (×10⁹ tu/ml); or (c) 1.5, 2.0, 2.25, 2.5, 2.75, 3.0, 3.25, 3.5, 4.0, 4.5, 5, 6, 7, 8, 8.4, 9, 9.3, 10, 15, 20, 25, or 50 (×10¹⁰ infectious units [iu]/ml).

Intracranial administration may be at any region in the brain and may encompass multiple regions when more than one intracranial delivery is administered. Such sites include, for example, in the brainstem (medulla and pons), mesencephalon, midbrain, cerebellum (including the deep cerebellar nuclei), diencephalon (thalamus, hypothalamus), telencephalon (corpus striatum, midbrain, cerebral cortex, or, within the cortex, the occipital, temporal, parietal or frontal lobes). Specific examples of intracranial injection sites are shown in Figure 1.

For identification of structures in the human brain, see, e.g., The Human Brain: Surface, Three-Dimensional Sectional Anatomy With MRI, and Blood Supply, 2nd ed., eds. Deuteron et al., Springer Vela, 1999; Atlas of the Human Brain, eds. Mai et al., Academic Press; 1997; and Co-Planar Sterotaxic Atlas of the Human Brain: 3-Dimensional Proportional System: An Approach to Cerebral Imaging, eds. Tamarack et al., Thyme Medical Pub., 1988. For identification of structures in the mouse brain, see, e.g., The Mouse Brain in Sterotaxic Coordinates, 2nd ed., Academic Press, 2000. If desired, the human brain structure can be correlated to similar structures in the brain of another mammal. For example, most mammals, including humans and rodents, show a similar topographical organization of the entorhinal-hippocampus projections, with neurons in the lateral part of both the lateral and medial entorhinal cortex projecting to the dorsal part or septal pole of the hippocampus, whereas the projection to the ventral hippocampus originates primarily from neurons in medial parts of the entorhinal cortex (Principles of Neural Science, 4th ed., eds Kandel et al., McGraw-Hill, 1991; The Rat Nervous System, 2nd ed., ed. Paxinos, Academic Press, 1995). Furthermore, layer II cells of the entorhinal cortex project to the dentate gyrus, and they terminate in the outer two-thirds of the molecular layer of the dentate gyrus. The axons from layer III cells project bilaterally to the cornu ammonis areas CA1 and CA3 of the hippocampus, terminating in the stratum lacunose molecular layer.

To deliver the vector specifically to a particular region of the central nervous system, especially to a particular region of the brain, it may be administered by sterotaxic microinjection. For example, on the day of surgery, patients will have the sterotaxic frame base fixed in place (screwed into the skull). The brain with sterotaxic frame base (MRI-compatible with fiduciary markings) will be imaged using high resolution MRI. The MRI images will then be transferred to a computer that runs stereotaxic software. A series of coronal, sagittal and axial images will be used to determine the target site of vector injection, and trajectory. The software directly translates the trajectory into 3-dimensional coordinates appropriate for the stereotaxic frame. Burr holes are drilled above the entry site and the stereotaxic apparatus localized with the needle implanted at the given depth. The vector in a pharmaceutically acceptable carrier will then be injected. The AAV vector is then administrated by direct injection to the primary target site and retrogradely transported to distal target sites via axons. Additional routes of administration may be used, e.g., superficial cortical application under direct visualization, or other non-stereotaxic application.

The total volume of material to be administered, and the total number of vector particles to be administered, will be determined by those skilled in the art based upon known aspects of gene therapy. Therapeutic effectiveness and safety can be tested in an appropriate animal model. For example, a variety of well-characterized animal models exist for LSDs, e.g., as described herein or in Watson et al. (2001) Methods Mol. Med., 76:383-403; or Jeyakumar et al. (2002) Neuropath. Appl. Neurobiol., 28:343-357; or in Metabolic and Molecular Bases of Inherited Disease, 8th edition (2001), published by McGraw-Hill.

In experimental mice, the total volume of injected AAV solution is for example, between 1 to 5 µl or about 3 µl per injection site, or alternatively between 10 to 400 µl or alternatively, between 100 to 400 µl, alternatively between 150 to 250 µl1 or alternatively about 200 µl. For other mammals, including the human brain, volumes and delivery rates are appropriately scaled. For example, it has been demonstrated that volumes of 150 µl can be safely injected in the primate brain (Janson et al. (2002) Hum. Gene Ther., 13:1391-1412). Treatment may consist of a single injection per target site, or may be repeated along the injection tract, if necessary. Multiple injection sites can be used. For example, in some embodiments, in addition to the first administration site, a composition comprising AAV carrying a transgene is administered to another site which can be contralateral or ipsilateral to the first administration site.

In the methods of the disclosure, AAV of any serotype can be used. In one instance or embodiment, AAV vectors capable of undergoing retrograde axonal transport may be used. The serotype of the viral vector may be selected from the group consisting from AAV1, AAV2, AAV3, AAV4, AAV5, AAV6, AAV7, and AAV8 (see, e.g., Gao et al. (2002) PNAS, 99:11854-11859; and Viral Vectors for Gene Therapy: Methods and Protocols, ed. Machida, Humana Press, 2003). Other serotype besides those listed herein can be used. Pseudotyped AAV vectors may also be used, which are those that comprise the ITRs of one AAV serotype in the capsid of a second AAV serotype; for example, an AAV vector that contains the AAV2 capsid and the AAV1 genome or an AAV vector that contains the AAV5 capsid and the AAV 2 genome. (Auricchio et al. (2001) Hum. Mol. Genet., 10(26):3075-81.)

AAV vectors are derived from single-stranded (ss) DNA parvoviruses that are nonpathogenic for mammals (reviewed in Muzyscka (1992) Curr. Top. Microb. Immunol., 158:97-129). Briefly, AAV-based vectors have the rep and cap viral genes that account for 96% of the viral genome removed, leaving the two flanking 145-basepair (bp) inverted terminal repeats (ITRs), which are used to initiate viral DNA replication, packaging and integration. In the absence of helper virus, wild-type AAV integrates into the human host-cell genome with preferential site-specificity at chromosome 19q 13.3 or it may remain expressed episomally. A single AAV particle can accommodate up to 5 kb of ssDNA, therefore leaving about 4.5 kb for a transgene and regulatory elements, which is typically sufficient. However, trans-splicing systems as described, for example, in United States Patent No. 6,544,785, may nearly double this limit.

In an illustrative embodiment, the AAV is AAV2 or AAV8. Adeno-associated virus of many serotypes, such as AAV2, have been extensively studied and characterized as gene therapy vectors. Those skilled in the art will be familiar with the preparation of functional AAV-based gene therapy vectors. Numerous references to various methods of AAV production, purification and preparation for administration to human subjects can be found in the extensive body of published literature (see, e.g., Viral Vectors for Gene Therapy: Methods and Protocols, ed. Machida, Humana Press, 2003). Additionally, AAV-based gene therapy targeted to cells of the CNS has been described in United States Patent Nos. 6,180,613 and 6,503,888.

The level of transgene expression in eukaryotic cells may be regulated by the transcriptional promoter and/or enhancer(s) within the transgene expression cassette. Tissue-specific promoters, such as liver-specific promoters, may be used in some embodiments. Tissue-specific enhancers, such as liver-specific enhancers, may be used in some embodiments. Combinations of tissue-specific promoters and tissue-specific enhancers, such as liver-specific promoters and enhancers may be used in the practice of the instant invention.

Non-limiting examples of promoters include, but are not limited to, the cytomegalovirus (CMV) promoter (Kaplitt et al. (1994) Nat. Genet. 8:148-154), CMV/human β3-globin promoter (Mandel et al. (1998) J. Neurosci. 18:4271-4284), GFAP promoter (Xu et al. (2001) Gene Ther., 8:1323-1332), the 1.8-kb neuron-specific enolase (NSE) promoter (Klein et al. (1998) Exp. Neurol. 150:183-194), chicken beta actin (CBA) promoter (Miyazaki (1989) Gene 79:269-277) and the β-glucuronidase (GUSB) promoter (Shipley et al. (1991) Genetics 10:1009-1018), the human serum albumin promoter, the alpha-1-antitrypsin promoter. To improve expression, other regulatory elements may additionally be operably linked to the transgene, such as, e.g., the Woodchuck Hepatitis Virus Post-Regulatory Element (WPRE) (Donello et al. (1998) J. Virol. 72: 5085-5092) or the bovine growth hormone (BGH) polyadenylation site.

Additional promoters which are suitable for the present invention may be any strong constitutive promoter which is capable of promoting expression of an associated coding DNA sequence in the liver. Such strong constitutive promoters include the human and murine cytomegalovirus promoter, truncated CMV promoters, human serum albumin promoter [HSA] and the alpha-1-antitrypsin promoter.

Liver-specific enhancer elements useful for the present invention may be any liver-specific enhancer that is capable of enhancing tissue-specific expression of an associated coding DNA sequence in the liver. Such liver-specific enhancers include one or more human serum albumin enhancers (HSA), human prothrombin enhancers (HPrT), alpha-1 microglobulin enhancers (A1MB), and intronic aldolase enhancers. Multiple enhancer elements may be combined in order to achieve higher expression. For example, two identical enhancers may be combined with a liver-specific promoter.

Viral vectors comprising the following promoter/ enhancer combinations may be used in the practice of the instant invention: one or more HSA enhancers in combination with either a CMV promoter or an HSA promoter; one or more enhancer selected from the group consisting of the human prothrombin (HPrT) enhancer and the alpha-1 microglobulin A1MB enhancer) in combination with a CMV promoter; and one or more enhancer elements selected from the group consisting HPrT enhancers of and A1MB enhancers, in combination with an a-1-antitrypsin promoter.

Additional information regarding liver-specific constructs are described in PCT Published Application No.: WO 01/36620. Alternatively, neuronal specific promoters are/or enhancers are useful for targeted delivery and expression of transgenes in the CNS.

In some aspects, it will be desirable to control transcriptional activity. To this end, pharmacological regulation of gene expression with AAV vectors can been obtained by including various regulatory elements and drug-responsive promoters as described, for example, in Habermaet al. (1998) Gene Ther., 5:1604-16011; and Ye et al. (1995) Science 283:88-91.

AAV preparations can be produced using techniques known in the art, e.g., as described in United States Patent No. 5,658,776 and Viral Vectors for Gene Therapy: Methods and Protocols, ed. Machida, Humana Press, 2003.

In certain aspects, detection of and/or the level of expression of the transgene may be desired. Method to detect gene expression are known in the art and can easily be applied as discussed infra, or modified by those of skill in the art.

Various methods are known for quantifying the expression of a gene of interest and include but are not limited to hybridization assays (Northern blot analysis) and PCR based hybridization assays.

In assaying for an alteration in mRNA level, the nucleic acid contained in a sample is first extracted according to a standard method in the art. For instance, mRNA can be isolated using various lytic enzymes or chemical solutions according to the procedures set forth in Sambrook et al. (1989), supra or extracted by nucleic-acid-binding resins following the accompanying instructions provided by the manufacturers.

Nucleic acid molecules having at least 10 nucleotides and exhibiting sequence complementarity or homology to the expression product can be used as hybridization probes or PCR primers. It is known in the art that a "perfectly matched" probe is not needed for a specific hybridization. Minor changes in probe sequence achieved by substitution, deletion or insertion of a small number of bases do not affect the hybridization specificity. In general, as much as 20% base-pair mismatch (when optimally aligned) can be tolerated. For example, a probe useful for detecting mRNA is at least about 80% identical to the homologous region of comparable size contained in a previously identified sequence, e.g., ASM sequence. Alternatively, the probe is at least 85% or even at least 90% identical to the corresponding gene sequence after alignment of the homologous region. The total size of fragment, as well as the size of the complementary stretches, will depend on the intended use or application of the particular nucleic acid segment. Smaller fragments of the gene will generally find use in hybridization, wherein the length of the complementary
region may be varied, such as between about 10 and about 100 nucleotides, or even full length according to the complementary sequences one wishes to detect.

Nucleotide probes having complementary sequences over stretches greater than about 10 nucleotides in length will increase stability and selectivity of the hybrid, and thereby improving the specificity of particular hybrid molecules obtained. One can design nucleic acid molecules having gene-complementary stretches of more than about 25 and even more preferably more than about 50 nucleotides in length, or even longer where desired. Such fragments may be readily prepared by, for example, directly synthesizing the fragment by chemical means, by application of nucleic acid reproduction technology, such as the PCR™ technology with two priming oligonucleotides as described in U.S. Patent No. 4,603,102 or by introducing selected sequences into recombinant vectors for recombinant production.

In certain embodiments, it will be advantageous to employ nucleic acid sequences of the present disclosure in combination with an appropriate means, such as a label, for detecting hybridization and therefore complementary sequences. A wide variety of appropriate indicator means are known in the art, including fluorescent, radioactive, enzymatic or other ligands, such as avidin/biotin, which are capable of giving a detectable signal. A fluorescent label or an enzyme tag, such as urease, alkaline phosphatase or peroxidase, instead of radioactive or other environmental undesirable reagents can also be used. In the case of enzyme tags, colorimetric indicator substrates are known which can be employed to provide a means visible to the human eye or spectrophotometrically, to identify specific hybridization with complementary nucleic acid-containing samples.

Hybridization reactions can be performed under conditions of different "stringency". Relevant conditions include temperature, ionic strength, time of incubation, the presence of additional solutes in the reaction mixture such as formamide, and the washing procedure. Higher stringency conditions are those conditions, such as higher temperature and lower sodium ion concentration, which require higher minimum complementarity between hybridizing elements for a stable hybridization complex to form. Conditions that increase the stringency of a hybridization reaction are widely known and published in the art. See, Sambrook, et al. (1989) supra.

One can also utilize detect and quantify mRNA level or its expression using quantitative PCR or high throughput analysis such as Serial Analysis of Gene Expression (SAGE) as described in Velculescu, V. et al. (1995) Science 270:484-487. Briefly, the method comprises isolating multiple mRNAs from cell or tissue samples suspected of containing the transcript. Optionally, the gene transcripts can be converted to cDNA. A sampling of the gene transcripts are subjected to sequence-specific analysis and quantified. These gene transcript sequence abundances are compared against reference database sequence abundances including normal data sets for diseased and healthy patients.

Probes also can be attached to a solid support for use in high throughput screening assays using methods known in the art. International PCT Application No. WO 97/10365 and U.S. Patent numbers 5,405,783, 5,412,087 and 5,445,934, for example, disclose the construction of high density oligonucleotide chips which can contain one or more sequences. The chips can be synthesized on a derivatized glass surface using the methods disclosed in U.S. Patent Nos. 5,405,783; 5,412,087 and 5,445,934. Photoprotected nucleoside phosphoramidites can be coupled to the glass surface, selectively deprotected by photolysis through a photolithographic mask, and reacted with a second protected nucleoside phosphoramidite. The coupling/deprotection process is repeated until the desired probe is complete.

The expression level of the gene can be determined through exposure of a sample suspected of containing the polynucleotide to the probe-modified chip. Extracted nucleic acid is labeled, for example, with a fluorescent tag, preferably during an amplification step. Hybridization of the labeled sample is performed at an appropriate stringency level. The degree of probe-nucleic acid hybridization is quantitatively measured using a detection device, such as a confocal microscope. See, U.S. Patent Nos. 5,578,832 and 5,631,734. The obtained measurement is directly correlated with gene expression level.

Hybridized probe and sample nucleic acids can be detected by various methods known in the art. For example, the hybridized nucleic acids can be detected by detecting one or more labels attached to the sample nucleic acids. The labels can be incorporated by any of a number of means known to those of skill in the art. In one aspect, the label is simultaneously incorporated during the amplification step in the preparation of the sample nucleic acid. Thus, for example, polymerase chain reaction (PCR) with labeled primers or labeled nucleotides will provide a labeled amplification product. In a separate instance , transcription amplification, as described above, using a labeled nucleotide (e.g., fluorescein-labeled UTP and/or CTP) incorporates a label in to the transcribed nucleic acids.

Alternatively, a label may be added directly to the original nucleic acid sample (e.g., mRNA, polyA, mRNA, cDNA, etc.) or to the amplification product after the amplification is completed. Means of attaching labels to nucleic acids are known to those of skill in the art and include, for example nick translation or end-labeling (e.g., with a labeled RNA) by kinasing of the nucleic acid and subsequent attachment (ligation) of a nucleic acid linker joining the sample nucleic acid to a label (e.g., a fluorophore).

Detectable labels suitable for use in the present disclosure include any composition detectable by spectroscopic, photochemical, biochemical, immunochemical, electrical, optical or chemical means. Useful labels in the present invention include biotin for staining with labeled streptavidin conjugate, magnetic beads (e.g., Dynabeads™), fluorescent dyes (e.g., fluorescein, Texas red, rhodamine, green fluorescent protein, and the like), radiolabels (e.g., ³H, ¹²⁵I, ³⁵S, ¹⁴C, or ³²P) enzymes (e.g., horseradish peroxidase, alkaline phosphatase and others commonly used in an ELISA), and colorimetric labels such as colloidal gold or colored glass or plastic (e.g., polystyrene, polypropylene, latex, etc.) beads. Patents teaching the use of such labels include U.S. Patents Nos. 3,817,837; 3,850,752; 3,939,350; 3,996,345; 4,277,437; 4,275,149; and 4,366,241.

Means of detecting such labels are known to those of skill in the art. Thus, for example, radiolabels may be detected using photographic film or scintillation counters, fluorescent markers can be detected using a photodetector to detect emitted light. Enzymatic labels are typically detected by providing the enzyme with a substrate and detecting the reaction product produced by the action of the enzyme on the substrate, and colorimetric labels are detected by simply visualizing the colored label.

Patent Publication WO 97/10365 describes methods for adding the label to the target (sample) nucleic acid(s) prior to or alternatively, after the hybridization. These are detectable labels that are directly attached to or incorporated into the target (sample) nucleic acid prior to hybridization. In contrast, "indirect labels" are joined to the hybrid duplex after hybridization. Often, the indirect label is attached to a binding moiety that has been attached to the target nucleic acid prior to the hybridization. Thus, for example, the target nucleic acid may be biotinylated before the hybridization. After hybridization, an avidin-conjugated fluorophore will bind the biotin bearing hybrid duplexes providing a label that is easily detected. For a detailed review of methods of labeling nucleic acids and detecting labeled hybridized nucleic acids, see Laboratory Techniques in Biochemistry and Molecular Biology, Vol. 24: Hybridization with Nucleic Acid Probes, P. Tijssen, ed. Elsevier, N.Y. (1993).

The nucleic acid sample also may be modified prior to hybridization to the high density probe array in order to reduce sample complexity thereby decreasing background signal and improving sensitivity of the measurement using the methods disclosed in International PCT Application No. WO 97/10365.

Results from the chip assay are typically analyzed using a computer software program. See, for example, EP 0717 113 A2 and WO 95/20681. The hybridization data is read into the program, which calculates the expression level of the targeted gene(s). This figure is compared against existing data sets of gene expression levels for diseased and healthy individuals. A correlation between the obtained data and that of a set of diseased individuals indicates the onset of a disease in the subject patient.

One can also modify know immunoassays to detect and quantify expression. Determination of the gene product requires measuring the amount of immunospecific binding that occurs between an antibody reactive to the gene product. To detect and quantify the immunospecific binding, or signals generated during hybridization or amplification procedures, digital image analysis systems including but not limited to those that detect radioactivity of the probes or chemiluminescence can be employed.

Expression of a polypeptide product may also be detected using biochemical means as known in the art for the particular polypeptide expressed.

The following examples provide illustrative embodiments of the invention. One of ordinary skill in the art will recognize the numerous modifications and variations that may be performed.

Such modifications and variations are encompassed within the scope of the invention. The examples do not in any way limit the invention.

### EXAMPLES

### Titration of Recombinant Vectors

AAV vector titers can be measured according to genome copy number (genome particles per milliliter). Genome particle concentrations may be based on Taqman® PCR of the vector DNA as previously reported (Clark et al. (1999) Hum. Gene Ther. 10:1031-1039; Veldwijk et al. (2002) Mol. Ther. 6:272-278). Briefly, the AAV vector is treated with a DNAse solution to remove any contaminating DNA that may interfere with an accurate measurement of the viral DNA. The AAV vector is then treated with capsid digestion buffer (50mM Tris-HCI pH 8.0, 1.0 mM EDTA, 0.5% SDS, 1.0 mg/ml proteinase K) at 50°C for 1 hour to release vector DNA. DNA samples are put through a polymerase chain reaction (PCR) with primers that anneal to specific sequences in the vector DNA, such as the promoter region, transgene, or the poly A sequence. The PCR results are then quantified by a Real-time Taqman® software, such as that provided by the Perkin Elmer-Applied Biosystems (Foster City, CA) Prism 7700 Sequence Detector System.

Vectors carrying an assayable marker gene such as the β-galactosidase or green fluorescent protein gene (GFP) can be titered using an infectivity assay. Susceptible cells (e.g., HeLa, or COS cells) are transduced with the AAV and an assay is performed to determine gene expression such as staining of β-galactosidase vector-transduced cells with X-gal (5-bromo-4chloro-3-indolyl-β-D-galactopyranoside) or fluorescence microscopy for GFP-transduced cells. For example, the assay is performed as follows: 4 × 10⁴ HeLa cells are plated in each well of a 24-well culture plate using normal growth media. After attachment, i.e., about 24 hours later, the cells are infected with Ad 5 at a multiplicity of infection (MOI) of 10 and transduced with serial dilutions of the packaged vector and incubated at 37°C. One to three days later, before extensive cytopathic effects are observed, the appropriate assay is performed on the cells (e.g., X-gal staining or fluorescence microscopy). If a reporter gene such as β-galactosidase is used, the cells are fixed in 2% paraformaldehyde, 0.5% glutaraldehyde and stained for β-galactosidase activity using X-gal. Vector dilutions that give well-separated cells are counted. Each positive cell represents 1 transduction unit (tu) of vector.

The full-length human ASM cDNA was cloned into a plasmid containing ITRs from AAV serotype 2 and 8. Jin et al. (2002) J Clin Invest. 109:1183-1191. AAV8-hASM contained serotype 2-inverted terminal repeats (ITRS) and the human acid sphingomyelinase (hASM) cDNA under the control of the DC-190 liver-restricted promoter [Ziegler et al. (2004). Mol. Ther. 9:231-240]. AAV2-hASM contained serotype 2-inverted terminal repeats (ITRS) and the human acid sphingomyelinase (hASM) cDNA under the control of the CMV enhancer and chicken β-actin promoter. Both recombinant vectors were produced by triple plasmid co-transfection of human 293 cells and column-purified. The final titers of the AAV8-hASM and AAV2-hASM preparations were 5.0 × 10¹² genome copies (gc) per ml, as determined by TaqMan PCR of the bovine growth hormone polyadenylation signal sequence which each vector contains.

Hybrid vectors can be produced by triple transfection using a series of helper plasmids containing serotype specific capsid coding domains in addition to the AAV serotype replication genes. This strategy allows the packaging of AAV ITR vectors into each serotype-specific virion. Rabinowitz, et al. (2002) J Virol. 76:791-801. With this approach the hASM recombinant genome can be used to generate a series of pseudotyped rAAV-hASM vectors. Recombinant AAV vectors can be purified by ion-exchange chromatography. O'Riordan, et al. (2000) J Gene Med 2: 444-54. Recombinant AAV vectors can also be purified by CsCI centrifugation Rabinowitz et al. (2002) J. Urrol. 76:791-801. The final titer of AAV-ASM virion particles (DNAse-resistant particles) can be determined by TaqMan PCR of the CMV sequence. Clark et al. (1999) Hum. Gene Therapy 10:1031-1039.

### Combination Brain and Systemic Gene Therapy in ASMKO mice

Acid sphingomyelinase deficient mice (ASMKO), K. Horinouchi et al., Nat. Genet. 10 (1995), pp. 288-292, were treated as follows:
Group 1) mice were injected with 3 e11 gc of an AAV2/8 vector comprising two HPrT enhancers, a human serum albumin promoter, and a human ASM transgene via a tail vein injection at 4 weeks of age;
Group 2) mice were injected with 2 e11 gc comprising an enhancer, promoter, and a human ASM transgene via brain injection split between 8 sites in the brain at 6 weeks of age;
Group 3) mice were injected with 3 e11 gc of AAV2/8 vector comprising two HPrT enhancers, a human serum albumin promoter, and a human ASM transgene via a tail vein injection at 4 weeks of age and two weeks later the same mice were injected with 2 e11 gc of an AAV2 vector comprising an enhancer, promoter, and a human ASM transgene via brain injection split between 8 sites in the brain; and
Group 4) mice received no injections or sham injections with only vehicle.
Group 5) Non-ASMKO, or wild-type, mice received no injections.

All ASMKO mice that underwent stereotaxic surgery were injected into 8 regions of the brain with 3µl of AAV2-hASM (1.5 e10 gc) per site for a total of 24 µl (1.2 e11 gc) per brain. The sites injected in the right hemisphere were the hypothalamus (-0.50, -1.00 mm, -3.50 mm), hippocampus (-2.00 mm, -1.75 mm, - 1.75 mm), medulla (-6.00 mm, -1.50 mm, -3.75 mm) and cerebellum (-6.00 mm, - 1.50 mm, -2.25 mm); and the sites injected in the left hemisphere were the striatum (0.50 mm, 1.75 mm, -2.75 mm), motor cortex (0.50 mm, 1.75 mm, -1.25 mm), midbrain (-4.50 mm, 1.00 mm, -3.50 mm) and cerebellum (-6.00 mm, 1.50 mm, -2.25 mm). Injections were performed with a Hamilton syringe (Hamilton USA, Reno, NV) at a rate of 0.5 µl/min, and the needle was left in place for 2 minutes after each injection to minimize upward flow of viral solution upon raising the needle.

All untreated ASMKO mice, and ASMKO mice in the AAV2 brain- and AAV8 systemic-alone groups eventually became moribund. In contrast, ASMKO mice treated by brain and systemic combination injections did not achieve moribund status, but were nonetheless sacrificed at 54 weeks for comparative analysis. Animals were extensively perfused to remove all blood, and divided into biochemical and histological cohorts. In the biochemical cohort, the liver, lung, spleen and skeletal muscle were cut into two pieces; one piece was analyzed for hASM levels and the other for sphingomyelin storage. In the brain, the left and right hemispheres were separated and each hemisphere were further cut into five 2-mm slabs along the A-P axis. Slabs from the left hemisphere were analyzed for hASM protein and anti-hASM

Serum levels of acid sphingomyelinase (ASM) and anti-ASM serum antibody levels were measured throughout the study. The mice underwent various tests throughout the study: body weight evaluations, accelerating rotarod testing, rocking rotarod testing, and Barnes maze testing. Survival curve data was also collected throughout the study duration. Upon the death of the mice, tissues were collected and sphingomyelin levels were measured in the brains, livers, lungs, spleens, and muscle tissue of each mouse. Human ASM protein expression in the brain and liver were also qualitatively evaluated using immunohistochemistry. The study was terminated at 54 weeks; all mice from group 3 (combo) were alive and healthy when the study was terminated.

Serum levels of acid sphingomyelinase were quantitated by an enzyme-linked immunosorbent assay (ELISA) using polyclonal antibodies that had been generated specifically against the human enzyme. Figure 7 graphically shows ASM protein levels in the blood serum over time. ASM was also measured via immunohistochemistry in the brains and livers of mice at death. Positive ASM staining was observed in the brains of mice from groups 2 and 3, with qualitatively brighter staining observed in mice from group 3. Staining was observed in the striatum, hippocampus, midbrain, and cerebellum. No ASM staining was observed in the brains of mice from group 1 or in untreated ASMKO mice. Positive ASM staining was observed in the livers of mice from groups 1 and 3. No ASM staining was observed in livers of mice from group 2 or in untreated ASMKO mice.

Tissue sphingomyelin levels were quantitated as follows. Tissue extracts were prepared by homogenizing 10 to 50 mg of tissue in chloroform:methanol (1:2) and incubating at 37°C for 1 h. Following removal of the cell debris by centrifugation, the homogenates were extracted twice with water and the organic phase (containing the lipids) was transferred to clean glass tubes and then dried under nitrogen with heating at 37°C. The amount of sphingomyelin present in the extracts was determined indirectly using the Amplex Red sphingomyelinase assay kit (Molecular Probes). Extracts were treated with a fixed amount of exogenous bacterial sphingomyelinase from Bacillus cereus (Sigma-Aldrich, St. Louis, MO, USA) in the Amplex Red working solution. Sphingomyelin was hydrolyzed by the bacterial enzyme to yield ceramide and phosphorylcholine. The latter was further hydrolyzed to choline, which in turn was oxidized to betaine and hydrogen peroxide. The released hydrogen peroxide was quantitated by reacting with Amplex Red to generate a highly fluorescent resorufin that could be detected by fluorescence emission at 590 nm. Normal sphingomyelin levels in the tissues of C57BL/6 mice are approximately 5-10% of those observed in age-matched ASMKO mice. Figures 8 and 9 graphically show the levels of sphingomyelin in the brain and visceral organs.

Levels of anti-human acid sphingomyelinase specific antibodies in the serum were determined by ELISA. See Figure 7 E. Serial dilutions of serum were added to wells of a 96-well plate coated with either the enzyme or heat-inactivated AAV particles. Bound antibodies were detected using horseradish peroxidase-conjugated goat anti-mouse immunoglobulin G (IgG), IgM, and IgA (Zymed, San Francisco, CA, USA). Plates were incubated with substrate (Sigma-Aldrich) for 20 min at room temperature for color development. Titers were defined as the reciprocal of the highest dilution of serum that produced an OD450 equal to or less than 0.1. Figures 7 A through 7 D graphically show levels of anti-hASM antibodies in circulation for treated and untreated mice.

Antibodies within the brain parenchyma were measured with a modified assay as follows. Tissue lysates were diluted 1:20 in antibody dilution buffer, and applied in duplicate to a 96-well plate coated with 100 ng hASM. The secondary antibody HRP conjugate and chromogenic substrate reactions were carried out as described above. The concentration of hASM-specific antibody bound should be directly correlated to the color intensity of the HRP reaction from the conjugate. Thus, the final data is reported as the absolute change in OD450 generated by the **1**:20 lysate dilution to provide a more sensitive determination of antibody levels compared to the tittering method used for serum.

Brain sections were analyzed for hASM expression using an anti-hASM biotinylated monoclonal antibody at a dilution of 1:200, and visualized with a streptavidin-Cy3-conjugated secondary antibody under red fluorescence [Passini, M.A. et al. (2005). Mol. Ther. 11:754-762]. Cholesterol substrate in the brain was detected using a filipin staining protocol as reported [Passini, M.A. et al. (2005). Mol. Ther. 11:754-762]. Briefly, filipin (Sigma, St. Louis, MO) was dissolved in 100% methanol to a working concentration of 10 mg/ml. Brain sections were incubated for 3 h at room temperature (RT) in the dark, followed by three washes at RT with PBS, and examined under blue fluorescence. Lysenin staining was done to determine the sphingomyelin substrate pattern *in situ,* as reported [Shihabuddin, L.S. et al. (2004). J. Neurosci. 24:10642-10651]. Briefly, lysenin (Peptides International, Louisville, Kentucky) as dissolved to 10 mg/ml in PBS containing 0.5% BSA, 0.02% saponin (Sigma), and 5% normal donkey serum. Brain sections were incubated with lysenin at 4°C overnight, followed by overnight incubation of 1:250 dilution of rabbit anti-lysenin antibody (Peptides International). Lysenin-positive cells were visualized with 1:250 dilution of FITC anti-rabbit antibody (Jackson ImmunoResearch, West Grove, PA) and examined using green fluorescence.

Each mouse was tested by accelerating and rocking rotarod for motor function on the Smartrod (AccuScan) using methods known in the art and reproduced in Sleat et al. (2004) J. Neurosci. 24:9117-9126. Mice were tested on the accelerating and rocking rotarod using the Smartrod Rotorod Program (AccuScan Instruments, Columbus, OH) to access motor function. The speed of the cylinder rotation on the accelerating rotarod was programmed to accelerate at a constant rate from 0-30 rpm over 60 seconds, and the rocking rotarod was programmed to accelerate forwards and backwards every 2.5 seconds to a final speed of 25 rpm over 54 seconds. Four trials were performed on each animal at each time point, and the latency to fall from the platform was recorded. The high latency score equates to good performance. Individual trials were separated by at least 15 min to allow the animal a rest period. Figures 3 and 4 graphically show the results of rotarod tests as a measurement of recovery of motor function.

Each mouse was tested using a Barnes Maze test. Mice were trained to locate a dark tunnel hidden beneath one of 20 holes positioned around the perimeter of a large, flat, plastic disk that was brightly illuminated by four overhead halogen lamps. The time to navigate through the maze to locate the correct, dark hole correlates to cognitive function inversely - a shorter navigation time is indicative of better cognitive function. Figure 5 graphically shows the results of Barnes Maze test as a measurement of recovery of cognitive function.

Survival curves are graphically shown in Figure 6.

A combination injection protocol of AAVhASM that targets both the brain and viscera was evaluated in addressing the functional abnormalities and disease sequelae of the ASMKO mouse. In the combination group (n = 11), ASMKO mice at 4 weeks of age received 3.0 x 10¹¹ genome copies (gc) of AAV8-hASM via tail vein injection. Two weeks later, at 6 weeks of age, the same mice were injected with AAV2-hASM into the motor cortex, striatum, midbrain, and cerebellum of the left hemisphere, and into the hypothalamus, hippocampus, medulla, and cerebellum of the right hemisphere. Each structure was injected with 1.5 X 10¹⁰ gc for a total of 1.2 X 10¹¹ gc per brain. The treated control groups received only systemic injections of AAV8-hASM at 4 weeks of age (n = 12) or only brain injections of AAV2-hASM at 6 weeks of age (n = 14), and the untreated control groups included ASMKO (n = 23) and wild type (n = 10) mice.

Periodic eye bleeds were performed to measure the levels of hASM and anti-hASM antibodies in circulation. Analysis of the serum from ASMKO mice treated by systemic injection alone and by combination injections exhibited the highest levels of circulating hASM. Transduction and subsequent expression by AAV8-hASM was primarily hepatic-mediated because of the tropism of this viral serotype and the selection of a liver-restricted promoter (DC190) in the design of the expression cassette. Both groups attained peak levels of hASM at 2 weeks post-injection, which subsequently declined up to 10-fold over the period of the study. Serum from the untreated ASMKO and AAV2-brain alone groups did not exhibit detectable levels of hASM. Further analysis of blood serum showed that the levels of anti-hASM antibody in the mice treated by combination or AAV8 systemic-alone injections were similar to the low baseline level observed in untreated ASMKO mice. In contrast, mice from the AAV2 brain-alone group exhibited a rapid and robust (200-fold increase) induction of anti-hASM antibody titers. Hence, mice treated by systemic injection of AAV8-hASM appear to be immunotolerized to the expressed hASM.

High levels of the enzyme were evident in the liver, lung, spleen and muscle of mice treated by the combination or AAV8 systemic-alone injections, presumably following mannose 6-phosphate receptor-mediated endocytosis of the enzyme in circulation. Human ASM levels in the visceral organs from the AAV2 brain-alone group were not significantly elevated above those observed in the untreated ASMKO control mice. Analysis of the brain from the combination and AAV2 brain-alone groups showed high levels of hASM throughout the neuraxis. However, the combination group exhibited significantly higher levels of hASM compared to brain-alone treatment despite the use of the same recombinant AAV2 vector in both cohorts. The levels of hASM in brains of mice treated solely by systemic injection were low and comparable to untreatedASMKO mice. This indicated that the hepatic derived hASM in the circulation were not able to traverse the blood brain barrier into the CNS. Interestingly, the titer of anti-hASM antibody in brain homogenates was approximately 10-fold higher in the AAV2 brain alone group compared to the other groups, including the combination group. Thus, the expression levels in the brain was a reciprocal of the antibody titers; the combination group showed high levels of hASM and low levels of anti-hASM antibodies, whereas the AAV2 brain-alone group showed low levels of hASM and high levels of anti-hASM antibodies.

The effect of hASM expression at correcting storage pathology in the viscera and brain of ASMKO Mice was determined. There was complete correction of sphingomyelin storage in all viscera tissues examined from the AAV8 systemic-alone and combination groups. In contrast, animals that received only brain injections contained high levels of sphingomyelin in the viscera that were similar to untreated ASMKO mice. Analysis of the sphingomyelin levels in the brain of ASMKO mice treated by combination injections showed global reduction of the substrate to wild-type levels. This was an improvement over the AAV2 brain-alone group, which exhibited a significant decrease of sphingomyelin only in the brain slabs corresponding to an injection site. Thus, the extent of correction in the brain-alone group was significantly less and never approached the efficacy observed in the combination group. The less-efficient reduction of sphingomyelin storage in the AAV2 brain-alone group correlated with the lower levels of enzyme observed in this group. A high level of brain sphingomyelin was observed in the AAV8 systemic-alone group that was similar to untreated ASMKO.

Brain sections were also analyzed histologically for hASM expression, and for sphingomyelin and cholesterol storage *in situ.* The pattern of hASM expression and clearance of sphingomyelin storage overlapped in the AAV2 brain-alone group. In contrast, the correction of sphingomyelin storage extended well beyond the injection sites in animals that received combination therapy. This produced both an overlapping and non-overlapping pattern of reversal of pathology compared to the areas of transduction with the combination therapy. This relationship was also observed with the cholesterol marker, filipin. Large regions of the brain were cleared of cholesterol storage in the combination group, whereas only local and more limited clearance of cholesterol was observed in the AAV2 brain-alone group. Hence, the ability of hASM to diffuse from the sites of injection to correct storage pathology in distal regions of the brain was significantly better in mice treated by combination injections compared to mice that received only brain injection. As may be expected from the biochemical data, the AAV8 systemic-alone group did not show any measurable correction of sphingomyelin or cholesterol storage in the brain.

Beginning at 10 weeks of age, mice were tested biweekly for motor function on the accelerating and rocking rotarods. Animals in the combination group showed significant improvements of motor performance on both rotarod tests at all time points examined (p <0.001). Mice treated by only AAV2 brain injections showed a modest improvement in motor performance on the accelerating rotarod at early time points when compared to the untreated ASMKO mice. However, their performance deteriorated at later time points demonstrating that brain-alone injections were not sufficient to sustain the correction in motor function. With the rocking rotarod, which is a more stringent test of motor function and coordination, the AAV2 brain-alone group performed poorly throughout the entire study. The AAV8 systemic-alone group showed little-to-no benefit on either rotatod test.

Beginning at 17 weeks of age, mice were also tested for cognitive function every 4 weeks on the Barnes maze. Mice utilized memory-mediated spatial navigation cues to escape a maze under the adverse light stimulus. The AAV2 brain-alone group performed better than untreated ASMKO, but never approached the performance level observed in wild type mice. Mice treated by systemic injections alone showed marginal improvement when compared to untreated controls. In contrast, the combination group performed on the Barnes maze with a proficiency that was similar to wild-type mice (p>0.05). Taken together with the rotarod data, correction of pathology in both the brain and viscera were necessary for the greatest functional outcome.

Mice were weighed every two weeks to evaluate their overall fitness, and their survival was analyzed by a Kaplan-Meier plot. The combination group had a weight gain profile that was similar to wild type animals, which was significantly better than the AAV2 brain- and AAV8 systemic-alone groups (p <0.001). Moribund animals, defined as heavy ataxia, inability to walk in a straight line without tumbling over, inability to groom, loss of 20% body weight and dehydration were sacrificed for humane purposes. The combination, AAV2 brain- and AAV8 systemic-alone groups displayed increased survival compared to untreated ASMKO mice, which had a median life-span of 34 weeks. However, all the ASMKO mice treated by combination therapy survived to 54 weeks of age and showed no signs of ataxia. This was a significant improvement over the AAV2 brain- and AAV8 systemic-alone groups, in which all the animals eventually became moribund with median life spans of 48 and 47 weeks (p <0.0001). None of the animals in the singly injected groups survived to 54 weeks. Thus, while treating only the brain or viscera did provide a significant survival benefit, this was less effective than treating both body compartments with combination therapy.

The specification is most thoroughly understood in light of the teachings of the references cited within the specification. The embodiments within the specification provide an illustration of embodiments of the invention and should not be construed to limit the scope of the invention. The skilled artisan readily recognizes that many other embodiments are encompassed by the invention.

To the extent the material referenced contradicts or is inconsistent with the present specification, the present specification will supercede any such material. The citation of any references herein is not an admission that such references are prior art to the present invention.

Unless otherwise indicated, all numbers expressing quantities of ingredients, cell culture, treatment conditions, and so forth used in the specification, including claims, are to be understood as being modified in all instances by the term "about." Accordingly, unless otherwise indicated to the contrary, the numerical parameters are approximations and may very depending upon the desired properties sought to be obtained by the present invention. Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the following claims.

## Claims

1. A first viral vector comprising a transgene encoding an immunogen; and
a second viral vector comprising a transgene encoding the immunogen for use in the treatment of lysosomal storage diseases with CNS involvement, wherein said treatment comprises the steps of:
a) administering said first viral vector to a mammal's liver tissue such that the mammal's brain is tolerised to the immunogen; and
b) subsequently administering said second viral vector to said mammal's brain
wherein said subsequent second viral vector administration occurs after said first viral vector comprising a transgene encoding the immunogen has been expressed in the liver of the mammal for an effective amount of time to generate tolerisation, and wherein the immunogen is the defective enzyme associated with the lysosomal storage disease being treated.

2. The first viral vector comprising a transgene encoding an immunogen and the second viral vector comprising a transgene encoding the immunogen for use in the method of treatment of claim 1, wherein said second vector is administered after expression of the transgene is detected in said mammal.

3. The first viral vector comprising a transgene encoding an immunogen and the second viral vector comprising a transgene encoding the immunogen for use in the method of treatment of claim 1, wherein the immunogen is an acid sphingomyelinase polypeptide or protein.

4. The first viral vector comprising a transgene encoding an immunogen and the second viral vector comprising a transgene encoding the immunogen for use in the method of treatment of claim 1, wherein the mammal is a human.

5. The first viral vector comprising a transgene encoding an immunogen and the second viral vector comprising a transgene encoding the immunogen for use in the method of treatment of claim 1, where the administration to the mammal's brain is a site selected from the group consisting of the brainstem, the midbrain, the hippocampus, the striatum, the medulla, the pons, the mesencephalon, the cerebellum, the thalamus, the hypothalamus, the cerebral cortex, the occipital lobe, the temporal lobe, the parietal lobe, and the frontal lobe.

6. The first viral vector comprising a transgene encoding an immunogen and the second viral vector comprising a transgene encoding the immunogen for use in the method of treatment of claim 1, wherein the administration to the mammal's brain is in the deep cerebellar nuclei of the cerebellum.

7. The first viral vector comprising a transgene encoding an immunogen and the second viral vector comprising a transgene encoding the immunogen for use in the method of treatment of claim 1, wherein the viral vector is an adeno associated virus (AAV).

8. The first viral vector comprising a transgene encoding an immunogen and the second viral vector comprising a transgene encoding the immunogen for use in the method of treatment of claim 7, wherein the AAV vector is selected from the group consisting of AAV1, AAV2, AAV3, AAV4, AAV5, AAV6, AAV7, and AAV8.

9. The first viral vector comprising a transgene encoding an immunogen and the second viral vector comprising a transgene encoding the immunogen for use in the method of treatment of claim 8, wherein the AAV is a recombinant AAV vector.

10. The first viral vector comprising a transgene encoding an immunogen and the second viral vector comprising a transgene encoding the immunogen for use in the method of treatment of claim 9, wherein the recombinant AAV vector is selected from the group consisting of AAV2/1, AAV2/2, AAV2/5, AAV2/7 and AAV2/8 serotype vectors.

11. The first viral vector comprising a transgene encoding an immunogen and the second viral vector comprising a transgene encoding the immunogen for use in the method of treatment of claim 9, wherein the first recombinant AAV vector comprises liver-specific enhancers and promoter elements.

12. The first viral vector comprising a transgene encoding an immunogen and the second viral vector comprising a transgene encoding the immunogen for use in the method of treatment of claim 1, wherein step b) is repeated.

13. A first viral vector comprising a transgene encoding an acid sphingomyelinase polypeptide or protein; and
a second viral vector comprising a transgene encoding an acid sphingomyelinase polypeptide or protein for use in the treatment of Niemann-Pick Type A disease in a mammal comprising the steps of:
a) administering said first viral vector to said mammal's liver tissue; and
b) subsequently administering said second viral vector to said mammal's brain,
wherein said subsequent second viral vector administration occurs after said first viral vector comprising a transgene encoding an acid sphingomyelinase polypeptide or protein has been expressed in the liver of the mammal for an effective amount of time to generate tolerisation.

14. The first viral vector comprising a transgene encoding an acid sphingomyelinase polypeptide or protein and the second viral vector comprising a transgene encoding an acid sphingomyelinase polypeptide or protein for use in the method of claim 13, wherein step b) is repeated.

15. The first viral vector comprising a transgene encoding an acid sphingomyelinase polypeptide or protein and the second viral vector comprising a transgene encoding an acid sphingomyelinase polypeptide or protein for use in the method of claim 13, wherein said second vector is administered after expression of the transgene is detected in said mammal.

16. The first viral vector comprising a transgene encoding an acid sphingomyelinase polypeptide or protein and the second viral vector comprising a transgene encoding an acid sphingomyelinase polypeptide or protein for use in the method of claim 13, wherein the mammal is a human.

17. The first viral vector comprising a transgene encoding an acid sphingomyelinase polypeptide or protein and the second viral vector comprising a transgene encoding an acid sphingomyelinase polypeptide or protein for use in the method of claim 13, where the administration to the mammal's brain is a site selected from the group consisting of the brainstem, the midbrain, the hippocampus, the striatum, the medulla, the pons, the mesencephalon, the cerebellum, the thalamus, the hypothalamus, the cerebral cortex, the occipital lobe, the temporal lobe, the parietal lobe, and the frontal lobe.

18. The first viral vector comprising a transgene encoding an acid sphingomyelinase polypeptide or protein and the second viral vector comprising a transgene encoding an acid sphingomyelinase polypeptide or protein for use in the method of claim 13, wherein the administration to the mammal's brain is in the deep cerebellar nuclei of the cerebellum.

19. The first viral vector comprising a transgene encoding an acid sphingomyelinase polypeptide or protein and the second viral vector comprising a transgene encoding an acid sphingomyelinase polypeptide or protein for use in the method of claim 13, wherein the viral vector is an adeno associated virus (AAV).

20. The first viral vector comprising a transgene encoding an acid sphingomyelinase polypeptide or protein and the second viral vector comprising a transgene encoding an acid sphingomyelinase polypeptide or protein for use in the method of claim 19, wherein the AAV vector is selected from the group consisting of AAV1, AAV2, AAV3, AAV4, AAV5, AAV6, AAV7, and AAV8.

21. The first viral vector comprising a transgene encoding an acid sphingomyelinase polypeptide or protein and the second viral vector comprising a transgene encoding an acid sphingomyelinase polypeptide or protein for use in the method of claim 19, wherein the AAV is a recombinant AAV vector.

22. The first viral vector comprising a transgene encoding an acid sphingomyelinase polypeptide or protein and the second viral vector comprising a transgene encoding an acid sphingomyelinase polypeptide or protein for use in the method of claim 21, wherein the recombinant AAV vector is selected from the group consisting of AAV2/1, AAV2/2, AAV2/5, AAV2/7 and AAV2/8 serotype vectors.

23. The first viral vector comprising a transgene encoding an acid sphingomyelinase polypeptide or protein and the second viral vector comprising a transgene encoding an acid sphingomyelinase polypeptide or protein for use in the method of claim 21, wherein the first recombinant AAV vector comprises liver-specific enhancers and promoter elements.

## Patentansprüche

1. Erster Virusvektor, umfassend ein Transgen, das für ein Immunogen codiert; und
zweiter Virusvektor, umfassend ein Transgen, das für das Immunogen codiert, zur Verwendung in der Behandlung von lysosomalen Speicherkrankheiten mit ZNS-Beteiligung, wobei die Behandlung die folgenden Schritte umfasst:
a) Verabreichen des ersten Virusvektors an das Lebergewebe eines Säugers, sodass das Hirn des Säugers gegenüber dem Immunogen tolerant gemacht wird; und
b) anschließendes Verabreichen des zweiten Virusvektors an das Hirn des Säugers, wobei die anschließende Verabreichung des zweiten Virusvektors stattfindet, nachdem der erste Virusvektor, umfassend ein Transgen, das für das Immunogen codiert, in der Leber des Säugers lang genug exprimiert worden ist, dass eine Toleranzbildung erfolgt ist, und wobei es sich bei dem Immunogen um das defekte Enzym handelt, das mit der behandelten lysosomalen Speicherkrankheit assoziiert ist.

2. Erster Virusvektor, umfassend ein Transgen, das für ein Immunogen codiert und zweiter Virusvektor, umfassend ein Transgen, das für das Immunogen codiert, zur Verwendung in dem Behandlungsverfahren nach Anspruch 1, wobei der zweite Vektor verabreicht wird, nachdem die Expression des Transgens in dem Säuger detektiert wurde.

3. Erster Virusvektor, umfassend ein Transgen, das für ein Immunogen codiert und zweiter Virusvektor, umfassend ein Transgen, das für das Immunogen codiert, zur Verwendung in dem Behandlungsverfahren nach Anspruch 1, wobei es sich bei dem Immunogen um ein Saure-Sphingomyelinase-Polypeptid oder -Protein handelt.

4. Erster Virusvektor, umfassend ein Transgen, das für ein Immunogen codiert und zweiter Virusvektor, umfassend ein Transgen, das für das Immunogen codiert, zur Verwendung in dem Behandlungsverfahren nach Anspruch 1, wobei es sich bei dem Säuger um einen Menschen handelt.

5. Erster Virusvektor, umfassend ein Transgen, das für ein Immunogen codiert und zweiter Virusvektor, umfassend ein Transgen, das für das Immunogen codiert, zur Verwendung in dem Behandlungsverfahren nach Anspruch 1, wobei die Verabreichung an das Hirn des Säugers eine Stelle aus der Gruppe bestehend aus dem Hirnstamm, dem Mittelhirn, dem Hippocampus, dem Striatum, der Medulla, dem Pons, dem Mesencephalon, dem Kleinhirn, dem Thalamus, dem Hypothalamus, der Hirnrinde, dem Okzipitallappen, dem Temporallappen, dem Parietallappen und dem Frontallappen ist.

6. Erster Virusvektor, umfassend ein Transgen, das für ein Immunogen codiert und zweiter Virusvektor, umfassend ein Transgen, das für das Immunogen codiert, zur Verwendung in dem Behandlungsverfahren nach Anspruch 1, wobei die Verabreichung an das Hirn des Säugers in den tiefen Kleinhirnkernen erfolgt.

7. Erster Virusvektor, umfassend ein Transgen, das für ein Immunogen codiert und zweiter Virusvektor, umfassend ein Transgen, das für das Immunogen codiert, zur Verwendung in dem Behandlungsverfahren nach Anspruch 1, wobei es sich bei dem Virusvektor um ein adenoassoziiertes Virus (AAV) handelt.

8. Erster Virusvektor, umfassend ein Transgen, das für ein Immunogen codiert und zweiter Virusvektor, umfassend ein Transgen, das für das Immunogen codiert, zur Verwendung in dem Behandlungsverfahren nach Anspruch 7, wobei der AAV-Vektor aus der Gruppe bestehend aus AAV1, AAV2, AAV3, AAV4, AAV5, AAV6, AAV7 und AAV8 ausgewählt ist.

9. Erster Virusvektor, umfassend ein Transgen, das für ein Immunogen codiert und zweiter Virusvektor, umfassend ein Transgen, das für das Immunogen codiert, zur Verwendung in dem Behandlungsverfahren nach Anspruch 8, wobei es sich bei dem AAV um einen rekombinanten AAV-Vektor handelt.

10. Erster Virusvektor, umfassend ein Transgen, das für ein Immunogen codiert und zweiter Virusvektor, umfassend ein Transgen, das für das Immunogen codiert, zur Verwendung in dem Behandlungsverfahren nach Anspruch 9, wobei der rekombinante AAV-Vektor aus der Gruppe bestehend aus AAV2/1-, AAV2/2-, AAV2/5-, AAV2/7- und AAV2/8-Serotyp-Vektoren ausgewählt ist.

11. Erster Virusvektor, umfassend ein Transgen, das für ein Immunogen codiert und zweiter Virusvektor, umfassend ein Transgen, das für das Immunogen codiert, zur Verwendung in dem Behandlungsverfahren nach Anspruch 9, wobei der erste rekombinante AAV-Vektor leberspezifische Enhancer- und Promotor-Elemente umfasst.

12. Erster Virusvektor, umfassend ein Transgen, das für ein Immunogen codiert und zweiter Virusvektor, umfassend ein Transgen, das für das Immunogen codiert, zur Verwendung in dem Behandlungsverfahren nach Anspruch 1, wobei Schritt b) wiederholt wird.

13. Erster Virusvektor, umfassend ein Transgen, das für ein Saure-Sphingomyelinase-Polypeptid oder -Protein codiert; und
zweiter Virusvektor, umfassend ein Transgen, das für ein Saure-Sphingomyelinase-Polypeptid oder -Protein codiert, zur Verwendung in der Behandlung von Niemann-Pick-Krankheit Typ A in einem Säuger, umfassend die folgenden Schritte:
a) Verabreichen des ersten Virusvektors an das Lebergewebe des Säugers; und
b) anschließendes Verabreichen des zweiten Virusvektors an das Hirn des Säugers, wobei die anschließende Verabreichung des zweiten Virusvektors stattfindet, nachdem der erste Virusvektor, umfassend ein Transgen, das für ein Saure-Sphingomyelinase-Polypeptid oder -Protein codiert, in der Leber des Säugers lang genug exprimiert worden ist, dass eine Toleranzbildung erfolgt ist.

14. Erster Virusvektor, umfassend ein Transgen, das für ein Saure-Sphingomyelinase-Polypeptid oder -Protein codiert; und zweiter Virusvektor, umfassend ein Transgen, das für ein Saure-Sphingomyelinase-Polypeptid oder -Protein codiert, zur Verwendung in dem Verfahren nach Anspruch 13, wobei Schritt b) wiederholt wird.

15. Erster Virusvektor, umfassend ein Transgen, das für ein Saure-Sphingomyelinase-Polypeptid oder -Protein codiert und zweiter Virusvektor, umfassend ein Transgen, das für ein Saure-Sphingomyelinase-Polypeptid oder -Protein codiert, zur Verwendung in dem Verfahren nach Anspruch 13, wobei der zweite Vektor verabreicht wird, nachdem die Expression des Transgens in dem Säuger detektiert worden ist.

16. Erster Virusvektor, umfassend ein Transgen, das für ein Saure-Sphingomyelinase-Polypeptid oder -Protein codiert und zweiter Virusvektor, umfassend ein Transgen, das für ein Saure-Sphingomyelinase-Polypeptid oder -Protein codiert, zur Verwendung in dem Verfahren nach Anspruch 13, wobei es sich bei dem Säuger um einen Menschen handelt.

17. Erster Virusvektor, umfassend ein Transgen, das für ein Saure-Sphingomyelinase-Polypeptid oder -Protein codiert und zweiter Virusvektor, umfassend ein Transgen, das für ein Saure-Sphingomyelinase-Polypeptid oder -Protein codiert, zur Verwendung in dem Verfahren nach Anspruch 13, wobei die Verabreichung an das Hirn des Säugers eine Stelle aus der Gruppe bestehend aus dem Hirnstamm, dem Mittelhirn, dem Hippocampus, dem Striatum, der Medulla, dem Pons, dem Mesencephalon, dem Kleinhirn, dem Thalamus, dem Hypothalamus, der Hirnrinde, dem Okzipitallappen, dem Temporallappen, dem Parietallappen und dem Frontallappen ist.

18. Erster Virusvektor, umfassend ein Transgen, das für ein Saure-Sphingomyelinase-Polypeptid oder -Protein codiert und zweiter Virusvektor, umfassend ein Transgen, das für ein Saure-Sphingomyelinase-Polypeptid oder -Protein codiert, zur Verwendung in dem Verfahren nach Anspruch 13, wobei die Verabreichung an das Hirn des Säugers in den tiefen Kleinhirnkernen erfolgt.

19. Erster Virusvektor, umfassend ein Transgen, das für ein Saure-Sphingomyelinase-Polypeptid oder -Protein codiert und zweiter Virusvektor, umfassend ein Transgen, das für ein Saure-Sphingomyelinase-Polypeptid oder -Protein codiert, zur Verwendung in dem Verfahren nach Anspruch 13, wobei es sich bei dem Virusvektor um ein adenoassoziiertes Virus (AAV) handelt.

20. Erster Virusvektor, umfassend ein Transgen, das für ein Saure-Sphingomyelinase-Polypeptid oder -Protein codiert und zweiter Virusvektor, umfassend ein Transgen, das für ein Saure-Sphingomyelinase-Polypeptid oder -Protein codiert, zur Verwendung in dem Verfahren nach Anspruch 19, wobei der AAV-Vektor aus der Gruppe bestehend aus AAV1, AAV2, AAV3, AAV4, AAV5, AAV6, AAV7 und AAV8 ausgewählt ist.

21. Erster Virusvektor, umfassend ein Transgen, das für ein Saure-Sphingomyelinase-Polypeptid oder -Protein codiert und zweiter Virusvektor, umfassend ein Transgen, das für ein Saure-Sphingomyelinase-Polypeptid oder -Protein codiert, zur Verwendung in dem Verfahren nach Anspruch 19, wobei es sich bei dem AAV um einen rekombinanten AAV-Vektor handelt.

22. Erster Virusvektor, umfassend ein Transgen, das für ein Saure-Sphingomyelinase-Polypeptid oder -Protein codiert und zweiter Virusvektor, umfassend ein Transgen, das für ein Saure-Sphingomyelinase-Polypeptid oder -Protein codiert, zur Verwendung in dem Verfahren nach Anspruch 21, wobei der rekombinante AAV-Vektor aus der Gruppe bestehend aus AAV2/1-, AAV2/2-, AAV2/5-, AAV2/7- und AAV2/8-Serotyp-Vektoren ausgewählt ist.

23. Erster Virusvektor, umfassend ein Transgen, das für ein Saure-Sphingomyelinase-Polypeptid oder -Protein codiert und zweiter Virusvektor, umfassend ein Transgen, das für ein Saure-Sphingomyelinase-Polypeptid oder -Protein codiert, zur Verwendung in dem Verfahren nach Anspruch 21, wobei der erste rekombinante AAV-Vektor leberspezifische Enhancer- und Promotor-Elemente umfasst.

## Revendications

1. Premier vecteur viral comprenant un transgène, qui code pour un immunogène ; et
deuxième vecteur viral comprenant un transgène, qui code pour l'immunogène, destinés à être utilisés dans le traitement de maladies associées au stockage lysosomal à implication du SNC, ledit traitement comprenant les étapes consistant à :
a) administrer ledit premier vecteur viral à un tissu du foie d'un mammifère, de sorte que le cerveau du mammifère soit rendu tolérant à l'immunogène ; et
b) administrer par la suite ledit deuxième vecteur viral audit cerveau du mammifère, ladite administration ultérieure du deuxième vecteur viral ayant lieu après que ledit premier vecteur viral, qui comprend un transgène codant pour l'immunogène, a été exprimé dans le foie du mammifère pendant une durée efficace pour générer une tolérance, et l'immunogène étant l'enzyme défectueuse associée à la maladie de stockage lysosomal étant traitée.

2. Premier vecteur viral comprenant un transgène, qui code pour un immunogène et deuxième vecteur viral comprenant un transgène, qui code pour l'immunogène, destinés à être utilisés dans le procédé de traitement de la revendication 1, ledit deuxième vecteur étant administré après qu'une expression du transgène est détectée dans ledit mammifère.

3. Premier vecteur viral comprenant un transgène, qui code pour un immunogène et deuxième vecteur viral comprenant un transgène, qui code pour l'immunogène, destinés à être utilisés dans le procédé de traitement de la revendication 1, l'immunogène étant un polypeptide ou une protéine de la sphingomyélinase acide.

4. Premier vecteur viral comprenant un transgène, qui code pour un immunogène et deuxième vecteur viral comprenant un transgène, qui code pour l'immunogène, destinés à être utilisés dans le procédé de traitement de la revendication 1, le mammifère étant un être humain.

5. Premier vecteur viral comprenant un transgène, qui code pour un immunogène et deuxième vecteur viral comprenant un transgène, qui code pour l'immunogène, destinés à être utilisés dans le procédé de traitement de la revendication 1, où l'administration au cerveau du mammifère est un site choisi dans le groupe constitué par le tronc cérébral, le cerveau moyen, l'hippocampe, les corps striés, la moelle, le pont, le mésencéphale, le cervelet, le thalamus, l'hypothalamus, le cortex cérébral, le lobe occipital, le lobe temporal, le lobe pariétal et le lobe frontal.

6. Premier vecteur viral comprenant un transgène, qui code pour un immunogène et deuxième vecteur viral comprenant un transgène, qui code pour l'immunogène, destinés à être utilisés dans le procédé de traitement de la revendication 1, l'administration au cerveau du mammifère étant dans les noyaux cérébelleux profonds du cervelet.

7. Premier vecteur viral comprenant un transgène, qui code pour un immunogène et deuxième vecteur viral comprenant un transgène, qui code pour l'immunogène, destinés à être utilisés dans le procédé de traitement de la revendication 1, le vecteur viral étant un virus adéno-associé (VAA).

8. Premier vecteur viral comprenant un transgène, qui code pour un immunogène et deuxième vecteur viral comprenant un transgène, qui code pour l'immunogène, destinés à être utilisés dans le procédé de traitement de la revendication 7, le vecteur VAA étant choisi dans le groupe constitué par les VAA1, VAA2, VAA3, VAA4, VAA5, VAA6, VAA7 et VAA8.

9. Premier vecteur viral comprenant un transgène, qui code pour un immunogène et deuxième vecteur viral comprenant un transgène, qui code pour l'immunogène, destinés à être utilisés dans le procédé de traitement de la revendication 8, le VAA étant un vecteur VAA recombinant.

10. Premier vecteur viral comprenant un transgène, qui code pour un immunogène et deuxième vecteur viral comprenant un transgène, qui code pour l'immunogène, destinés à être utilisés dans le procédé de traitement de la revendication 9, le vecteur VAA recombinant étant choisi dans le groupe constitué par les vecteurs des sérotypes VAA2/1, VAA2/2, VAA2/5, VAA2/7 et VAA2/8.

11. Premier vecteur viral comprenant un transgène, qui code pour un immunogène et deuxième vecteur viral comprenant un transgène, qui code pour l'immunogène, destinés à être utilisés dans le procédé de traitement de la revendication 9, le premier vecteur VAA recombinant comprenant des éléments amplificateurs et promoteurs spécifique du foie.

12. Premier vecteur viral comprenant un transgène, qui code pour un immunogène et deuxième vecteur viral comprenant un transgène, qui code pour l'immunogène, destinés à être utilisés dans le procédé de traitement de la revendication 1, l'étape b) étant répétée.

13. Premier vecteur viral comprenant un transgène, qui code pour un polypeptide ou une protéine de la sphingomyélinase acide ; et
deuxième vecteur viral comprenant un transgène, qui code pour un polypeptide ou une protéine de la sphingomyélinase acide, destinés à être utilisés dans le traitement de la maladie de Niemann-Pick de type A chez un mammifère, comprenant les étapes consistant à :
a) administrer ledit premier vecteur viral audit tissu hépatique du mammifère ; et
b) administrer par la suite ledit deuxième vecteur viral audit cerveau du mammifère, ladite administration ultérieure du deuxième vecteur viral ayant lieu après que ledit premier vecteur viral comprenant un transgène, qui code pour un polypeptide ou une protéine de la sphingomyélinase acide, a été exprimé dans le foie du mammifère pendant une durée efficace pour générer une tolérance.

14. Premier vecteur viral comprenant un transgène, qui code pour un polypeptide ou une protéine de la sphingomyélinase acide et deuxième vecteur viral comprenant un transgène, qui code pour un polypeptide ou une protéine de la sphingomyélinase acide, destinés à être utilisés dans le procédé de la revendication 13, l'étape b) étant répétée.

15. Premier vecteur viral comprenant un transgène, qui code pour un polypeptide ou une protéine de la sphingomyélinase acide et deuxième vecteur viral comprenant un transgène, qui code pour un polypeptide ou une protéine de la sphingomyélinase acide, destinés à être utilisés dans le procédé de la revendication 13, ledit deuxième vecteur étant administré après qu'une expression du transgène est détectée dans ledit mammifère.

16. Premier vecteur viral comprenant un transgène, qui code pour un polypeptide ou une protéine de la sphingomyélinase acide et deuxième vecteur viral comprenant un transgène, qui code pour un polypeptide ou une protéine de la sphingomyélinase acide, destinés à être utilisés dans le procédé de la revendication 13, le mammifère étant un être humain.

17. Premier vecteur viral comprenant un transgène, qui code pour un polypeptide ou une protéine de la sphingomyélinase acide et deuxième vecteur viral comprenant un transgène, qui code pour un polypeptide ou une protéine de la sphingomyélinase acide, destinés à être utilisés dans le procédé de la revendication 13, où l'administration au cerveau du mammifère est un site choisi dans le groupe constitué par le tronc cérébral, le cerveau moyen, l'hippocampe, les corps striés, la moelle, le pont, le mésencéphale, le cervelet, le thalamus, l'hypothalamus, le cortex cérébral, le lobe occipital, le lobe temporal, le lobe pariétal et le lobe frontal.

18. Premier vecteur viral comprenant un transgène, qui code pour un polypeptide ou une protéine de la sphingomyélinase acide et deuxième vecteur viral comprenant un transgène, qui code pour un polypeptide ou une protéine de la sphingomyélinase acide, destinés à être utilisés dans le procédé de la revendication 13, l'administration au cerveau du mammifère étant dans les noyaux cérébelleux profonds du cervelet.

19. Premier vecteur viral comprenant un transgène, qui code pour un polypeptide ou une protéine de la sphingomyélinase acide et deuxième vecteur viral comprenant un transgène, qui code pour un polypeptide ou une protéine de la sphingomyélinase acide, destinés à être utilisés dans le procédé de la revendication 13, le vecteur viral étant un virus adéno-associé (VAA).

20. Premier vecteur viral comprenant un transgène, qui code pour un polypeptide ou une protéine de la sphingomyélinase acide et deuxième vecteur viral comprenant un transgène, qui code pour un polypeptide ou une protéine de la sphingomyélinase acide, destinés à être utilisés dans le procédé de la revendication 19, le vecteur VAA étant choisi dans le groupe constitué par les VAA1, VAA2, VAA3, VAA4, VAA5, VAA6, VAA7 et VAA8.

21. Premier vecteur viral comprenant un transgène, qui code pour un polypeptide ou une protéine de la sphingomyélinase acide et deuxième vecteur viral comprenant un transgène, qui code pour un polypeptide ou une protéine de la sphingomyélinase acide, destinés à être utilisés dans le procédé de la revendication 19, le VAA étant un vecteur VAA recombinant.

22. Premier vecteur viral comprenant un transgène, qui code pour un polypeptide ou une protéine de la sphingomyélinase acide et deuxième vecteur viral comprenant un transgène, qui code pour un polypeptide ou une protéine de la sphingomyélinase acide, destinés à être utilisés dans le procédé de la revendication 21, le vecteur VAA recombinant étant choisi dans le groupe constitué par les vecteurs des sérotypes VAA2/1, VAA2/2, VAA2/5, VAA2/7 et VAA2/8.

23. Premier vecteur viral comprenant un transgène, qui code pour un polypeptide ou une protéine de la sphingomyélinase acide et deuxième vecteur viral comprenant un transgène, qui code pour un polypeptide ou une protéine de la sphingomyélinase acide, destinés à être utilisés dans le procédé de la revendication 21, le premier vecteur VAA recombinant comprenant des éléments amplificateurs et promoteurs spécifiques du foie.
